# EUROPEAN PATENT SPECIFICATION

(11) **EP 2 858 550 B1**
(45) Date of publication and mention of the grant of the patent: **10.06.2020**
(21) Application number: 13803956.5
(22) Date of filing: 12.06.2013
(51) Int. Cl.: G16H 10/00, A61B 5/00, G08B 25/08, G08B 21/00

(54) **A METHOD AND APPARATUS FOR FACILITATING THE MANAGEMENT OF HEALTH AND SECURITY**
VERFAHREN UND VORRICHTUNG ZUR ERLEICHTERUNG DER VERWALTUNG VON GESUNDHEIT UND SICHERHEIT
PROCÉDÉ ET APPAREIL POUR FACILITER LA GESTION DE LA SANTÉ ET DE LA SÉCURITÉ

(30) Priority: 12.06.2012 AU 2012902447; 26.11.2012 AU 2012905155; 12.04.2013 AU 2013204692
(43) Date of publication of application: 15.04.2015
(73) Proprietor: C. Rafin & Co Pty Ltd, Wollongong New South Wales 2500 (AU)
(72) Inventor: RAFIN, Joshua, Cordeaux Heights NSW 2526 (AU); STAWSKI, Victor Belina, Cringila NSW 2502 (AU); CELIMA, Massimo Feliciano, Shell Cove NSW 2529 (AU); RAFIN, Claudio Giuseppe, Cordeaux Heights NSW 2526 (AU)
(74) Representative: De Clercq, Ann G. Y.
(86) International application number: PCT/AU2013/000626
(87) International publication number: WO 2013/185176

(56) References cited:
- US-A1- 2006 154 642
- US-A1- 2012 095 352
- US-A1- 2012 095 352
- LITZ L ET AL: "Covering Assisted Living Key Areas based on Home Automation Sensors", NETWORKING, SENSING AND CONTROL, 2007 IEEE INTERNATIONAL CONFERENCE ON, IEEE, PI, 1 April 2007 (2007-04-01), pages 639-643, XP031178385, ISBN: 978-1-4244-1075-0

## Description

### Field of the Invention

The present invention relates to a method and apparatus for facilitating the management of health and security, and, particularly, but not exclusively, to a method and apparatus of facilitating the management of health and security remotely for patients at home.

### Background of the Invention

Health management has traditionally been carried out in institutions such as hospitals. There is an increasing trend today, however, particularly in developed countries where there is an aging demographic, to managing people in their own homes. Home care may be facilitated by organisations that provide care in the community. This may be done by the provision of medical professionals, such as community nurses, who visit patients at home.

It is also known to use technology to facilitate personal security in the home, particularly for aged, infirm and disabled persons. For example, "personal alarm" devices are available to enable a person to alert a remote operator if they are in an alarm situation. For example, they may have fallen down and been hurt, and they may alert the remote operator by actuating their alarm device (which may be a wearable device).

Such Personal Emergency Response Systems (PERS) are implemented using hardware specifically designed for the PERS function, usually separate from any other security systems or any other systems in the home/residence. There are in the order of half a million PERS's currently in Australia, each having purpose-designed hardware and software.

Management of health care issues (e.g. monitoring of health parameters such as blood pressure) are generally reliant on regular interaction with health professionals, such as visits to the patient's residence by community nurses.

So called "tele health care" technology has been developed in some areas to remotely facilitate treatment. Such systems are generally the provenance of major technology companies, however, and have been designed particularly for "high end" health applications, such as facilitating remote surgery so that, for example, a surgeon can manage an operation in a hospital from a remote location. These tele health care systems are complex and expensive. No satisfactory system has been developed for facilitating health care in the community residence of a patient so that health care services can be carried out remotely, and the patient can be maintained in their home rather than having to enter an institution.

US 2006/154642 A1, considered by the Examining Division as the closest prior art, discloses systems and apparatuses including devices, biosensors, environmental sensors, security related sensors, networked products, communications processors and components, alert and information components, processors, and software to support: 1) facilitating medication regimen and patient/user health administration, dosage control, tracking, compliance, information inquiry and presentation, reminder and notification; 2) providing monitoring, information, ordering, and intervention; 3) presenting the option of leveraging the preventative care, alert and notification components with other components to facilitate user or occupant well being, along with living, work area and dwelling environmental or security safety; and 4) enhancing the dwelling, living or work area with products that may be networked to support the widespread acceptance of these systems and apparatuses.

US 2012/095352 A1 discloses a heart monitoring system for a person, which includes one or more wireless nodes forming a wireless network; a wearable sensor having a wireless transceiver adapted to communicate with the one or more wireless nodes; and a software module receiving data from the wireless nodes to detect changes in patient vital signs.

Litz et al: 'Covering Assisted Living Key Areas based on Home automation Sensors', Networking, Sensing and control, 2007 IEEE International Conference on, IEEE, PI, 1 April 2007, pages 639-643, XP031178385, ISBN 978-1-4244-1075-0 discloses an assisted living system for elderly people. The assisted living key areas such as comfort, safety and health are discussed. A strategy is elaborated which mainly uses standard home automation sensors. The solution is based on the transformation of sensor signals by automata and fuzzy rules to detect safety-or health-critical situations.

A problem to resolve when implementing technology in a home environment, such as health monitoring or security, is that old, infirm, disabled people may not be comfortable with the use of "modern" computing technology. In particular, they may find computing interfaces complex and difficult to deal with.

### Summary of Invention

In accordance with a first aspect, the present invention provides a health and security management apparatus for facilitating the management of health and security of a patient and their home, the management apparatus comprising a processing device having a processor, memory, a remote communications interface for communicating with a remote monitoring system which is arranged for communications with a plurality of the health and security apparatus, a local communications interface for communication with devices locally, a patient interface for operation by the patient, the patient interface comprising a display screen for display and input means for patient input, a health monitoring arrangement being arranged to obtain health data on patient health, and arranged to transmit the data via the remote communication interface to the remote monitoring system, a security monitoring arrangement arranged to obtain data on patient security, including personal alarm data and home security data, including obtaining the patient security data via the local communications interface from security devices locally, and arranged to transmit the data via the remote communications interface to the remote monitoring system, a home automation arrangement arranged to produce home automation data to control home automation devices in the patient's home, the home automation data being transmitted to the home automation devices via the local communication interface, and the home automation arrangement being arranged to receive home automation data from the remote monitoring system via the remote communications interface, for control of home automation devices from the remote monitoring system, wherein the remote communication interface comprises a plurality of different communication modules, enabling communications by different pathways, to enable redundancy of communications with the remote monitoring system, and wherein the health and security management apparatus converges the health monitoring arrangement, the security monitoring arrangement and the home automation arrangement into a single apparatus, wherein the single apparatus comprises a plurality of units arranged to be mounted to each other, and wherein the health and security management apparatus being arranged to port all health data, security data and home automation data.

In an embodiment, the health monitoring arrangement may be arranged to receive data from sensors, which are arranged to monitor patient health parameters. Such sensors may include blood pressure sensors, heart rate monitors, and other sensors. The health monitoring arrangement is arranged to obtain the health data produced by the sensors and the communications interface is arranged to transmit the data to a remote location, for remote monitoring of the patient parameters. A remotely located medical professional may be able to determine the health of the patient from monitored parameters, and take any action necessary.

The security monitoring arrangement may be arranged to receive signals from personal alarm device(s) which the patient may activate when an emergency situation arises. The security monitoring arrangement may then provide security data for communication to the remote location. For example, an alarm can then be communicated to the remote location for an operative at the remote location to take the appropriate action.

In an embodiment, the security monitoring arrangement may be arranged to receive signals from home security alarm(s) which may be activated on, for example, unauthorised entry to the home, smoke alarms, flood alarms, and other security monitoring apparatus. The security data may be provided accordingly to the remote location.

The health and security management apparatus of at least an embodiment of the invention therefore has the advantage that it converges security monitoring, health parameter monitoring and home automation control into a single system. The security and health of the patient can therefore be monitored remotely from their home. Advantageously, this may reduce the need for visits by medical professionals to take routine health parameter measurements, such as blood pressure, as well as maintaining security of the patient and enabling alarms to be delivered remotely when required. Advantageously, systems that are currently present in residences, which perform particular functions, such as PERS systems, may be replaced by a single, holistic health and security apparatus, for performing all functions for health and security of a patient.

The patient residence may be a domestic residence, or may be residential care, such as an apartment in a patient complex. For example, a retirement home. It may be any other patient residence.

In an embodiment, the management apparatus comprises a patient interface, enabling control of the apparatus by the patient. In an embodiment, the patient interface comprises a patient communications interface which is arranged to enable the patient to communicate remotely. The patient may communicate with medical professionals to advise them of health parameters, health matters and any other information, via the patient communication interface. Also to receive information from the medical professionals. In an embodiment, the patient communications interface comprises an audio and video interface.

In an embodiment, the home automation arrangement, is arranged to facilitate control of devices in the home, such as washing machines, televisions, refrigerators, air conditioning and others. The home automation arrangement may be arranged to interface with control sensors and control devices for controlling the devices in the home. The management apparatus in this embodiment therefore provides a single monitoring system that can be used for home automation, remote communications with a remote location for patient health monitoring, remote health parameter monitoring and security including personal emergency response systems. The home automation arrangement is arranged to be operated from the remote location. An operative from a remote location may, for example, be able to control the lighting, heating and other systems/devices at the patient's residence.

In an embodiment, the management apparatus comprises a portable unit, arranged to be carried around by the patient, and a dock unit. The portable unit is arranged to be docked with the dock unit.

In an embodiment, the portable unit and dock unit are arranged to be physically docked.

In an embodiment, the portable unit includes a screen interface, which may be a touch screen. The touch screen may be capable of rendering video. In an embodiment, the dock is arranged to support the portable unit in an upright position for ease of access by the patient.

In an embodiment, the video interface of the patient communications interface is via the video/touch screen of the portable device. The portable device may also include an audio arrangement for enabling the audio interface. In an embodiment the dock may include an audio arrangement, comprising speakers and microphones. In an embodiment, the dock audio arrangement may be an enhanced arrangement as compared with the audio arrangement of the portable device. The dock may therefore provide better audio facilities for use by the patient. In an embodiment, the dock can be used to implement enhanced audio facilities to cater for people with enhanced audio needs. For example, elderly patients may have problems with hearing which are not catered for by standard audio interfaces that may be found, for example, on standard tablet computing devices. Providing enhanced audio facilities in the dock addresses this problem.

In an embodiment, the communications interface may comprise one or more communications modules in the portable device and dock. The communications modules may cater for a plurality of different communication facilities, in order to allow for redundancy of communications. This is particularly important in an emergency response system, such as a PERS. In an embodiment, the communications modules may enable mobile communications (e.g. GSM), communications via the Internet, SMS communications, and others, enabling redundancy of communications to the remote location to ensure that any personal response alarm is transmitted. In an embodiment, the communications modules may also comprise WiFi facilities and Bluetooth™ to enable communications locally between the portable device and dock, and also between sensors/control devices in the residence and the management apparatus.

In an embodiment, both the dock unit and portable unit are electronic devices and require power to function. In an embodiment, the portable unit includes a rechargeable battery which is recharged when the portable unit is connected with the dock unit. In an embodiment, the dock unit includes a large capacity battery backup, in case power supply should fail. In an embodiment, the dock unit is arranged to be connected to a mains power supply and, when the portable unit is connected, the portable unit battery can be recharged from the mains. Also the large capacity battery backup of the dock can be recharged from the mains. In an embodiment, the large capacity battery is a sufficient capacity to satisfy relevant jurisdictional standards regarding power supply for emergency systems.

In an embodiment, the patient interface comprises a touch screen on the portable unit and a touch screen interface provided by an appropriately configured processor in the portable unit. In an embodiment, the dock mounts a plurality of user interface elements forming part of the patient interface. The user interface elements may be push buttons or touch buttons.

In an embodiment, the function of one or more of the buttons is configurable by the patient and/or by a medical professional. In an embodiment, the one or more buttons may be configured from a remote location.

Advantageously, providing a patient communications interface having simple interface elements, such as push buttons, that can be configured for particular functions, facilitates ease of use by elderly, infirm or disabled patients. The interface elements may be of a size which is easy to manipulate. For example, where the elements are push buttons, they may be relatively larger than standard push buttons on standard keyboards e.g. computer keyboards.

In an embodiment, the portable unit is a tablet computing device.

In an embodiment, the health and security management apparatus comprises a further portable unit. In an embodiment, the further portable unit is a telephone, such as a wireless telephone. A telephone is a familiar interface to elderly patients in particular. Further, providing telephone services via the health and security management apparatus and the communications device made available thereby, avoids the need to have a separate telephone service. Advantageously, all communications, health and security management services and other services, such as Internet and tv, may be provided by the health and security management apparatus. In an embodiment, the telephone may communicate with the portable unit via local wireless, such as Bluetooth™. The communications device will then enroute the communications from the telephone via remote communications networks, such as landline, mobile telephony or other remote communications networks.

The health and security management apparatus is arranged to communicate data with a monitoring system at the remote location. The monitoring system may comprise an appropriately configured computing system with communications gateways for facilitating communication with the health and security management apparatus. In an embodiment, the monitoring system is arranged for communications with a plurality of health and security management apparatus, so that a plurality of patients can be monitored.

In an embodiment, the monitoring system may also provide a medical carer administrative system, which is arranged for communications with a medical carer device. The medical carer device may be an appropriately configured tablet computer or a smartphone, which enables a medical carer to enter medical information regarding patients and/or receive and enter medical information, such as appointments with patients, location of the patients and other patient related information.

In an embodiment, the health and security management apparatus may be configured for operation by a plurality of patients. In one embodiment, for example, a family, such as a husband and wife, may have a single security management apparatus. They may have separate "logins" to the apparatus so that their health parameters and PERS systems can be partitioned, for example. Security alarms and home automation may not need to be partitioned.

In another embodiment, the health and security management apparatus may be configured for operation by a number of people. For example, the apparatus may be positioned in a community centre of a retirement village or the community. It may be accessed by the members of the retirement village. Each may have a separate login so that their health parameters can be managed separately, for example, via the health and security management apparatus "kiosk".

In accordance with a second aspect, the present description provides a monitoring system, for facilitating the management of the health and security of patients at remote locations, comprising a monitoring communications system for communicating with a health and security management apparatus in accordance with the first aspect of the invention, and being arranged to receive health data on patient health and security data on security of the patient.

In an embodiment, the monitoring system is arranged to provide control data for remote control of the management apparatus. Control data may be provided from the monitoring system for remote control of the patient's home systems. For example, door locks may be remotely controlled, lights may be remotely controlled, heaters may be remotely controlled. Anything connected to the home automation system may be remotely controlled from the monitoring system. This advantageously allows for control of home systems where a person is, for example, disabled or infirm and has difficulty dealing with the control themselves, or where the patient is in an emergency situation.

In an embodiment, the monitoring system also comprises a medical professional administrative system enabling logging of medical information regarding patients and administrative information, such as appointments of medical carers with patients.

In an embodiment, the medical carer administrative system further comprises an administrative database, hosted by the monitoring system for storing information relating to patients cared for and tasks carried out by the medical carer.

In accordance with a third aspect, the present invention provides a health management system, arranged for management of health and security of patients at a plurality of different locations, comprising a health and security management apparatus in accordance with the first aspect of the invention and a monitoring system in accordance with the second aspect of the description.

In accordance with a fifth aspect, the present description provides a computer program, comprising instructions for instructing a computer to implement a monitoring system in accordance with the second aspect of the description.

In accordance with a sixth aspect the present description comprises a computer readable medium, providing a computer program in accordance with the fifth aspect of the description.

In accordance with a seventh aspect, the present description provides a data signal, comprising a computer program in accordance with the fifth aspect of the present description.

In accordance with an eighth aspect, the present description provides a computer program, comprising instructions for instructing a computing apparatus to implement a health and security management apparatus in accordance with the first aspect of the description.

In accordance with a ninth aspect, the present description comprises a computer readable medium, providing a computer program in accordance with the eighth aspect of the description.

In accordance with a tenth aspect, the present description provides a data signal, comprising a computer program in accordance with the eighth aspect of the description.

### Brief description of the Figures

Features and advantages of the present invention will become apparent from the following description of embodiments thereof, by way of example only, with reference to the accompanying drawings in which;
Figure 1 is a schematic diagram illustrating a health and security monitoring system in accordance with an embodiment of the present invention;
Figure 2 is a schematic block diagram of a health and security management apparatus in accordance with an embodiment of the present invention.
Figure 3 is a further schematic diagram of the management apparatus of figure 2;
Figure 4 is yet a further schematic diagram of the management apparatus of Figure 2;
Figures 5-11 are various views of a management apparatus in accordance with an embodiment of the present invention;
Figure 12 is a flow diagram illustrating an example use implementation of a management system of an embodiment of the present invention;
Figures 13(a) to 13(d) are examples of interfaces provided by a management apparatus in accordance with an embodiment of the present invention, for the use implementation of figure 12;
Figure 14 is a flow diagram of a further use implementation for the monitoring system;
Figures 15(a) to 15(g) are examples of interfaces which may be used or generated by the monitoring system for the use implementation of figure 41;
Figure 16 is a flow diagram of a further use implementation of the management system;
Figures 17(a) to 17(c) are interfaces of the management system which may be generated for the use implementation of figure 16;
Figure 18 is a flow diagram illustrating a further use implementation of the management system;
Figures 19(a) to 19(u) are interfaces which may be provided by a medical carer apparatus for the use implementation of figure 18;
Figures 20(a) to 20(l) are further interfaces which may be generated by a medical carer apparatus of the monitoring system, for different operations;
Figures 21-24 are sample "screen shots" illustrating operation of an interface of a management apparatus in accordance with an embodiment of the present invention;
Figures 25(a) to 25(d) are further sample "screen shots" illustrating operation of a communications "widget" of an embodiment of the management apparatus;
Figure 26 is a perspective view of a further embodiment of a health and security management apparatus in accordance with the present invention:
Figure 27 is a block diagram illustrating functional components of a first arrangement for the embodiment of Figure 26;
Figure 28 is a block diagram illustrating functional components for a second arrangement of the embodiment of Figure 26, and;
Figures 29 to 40 are sample "screenshots" illustrating operation of an interface of a management apparatus in accordance with an embodiment of the present invention.

### Detailed Description of embodiments of the invention

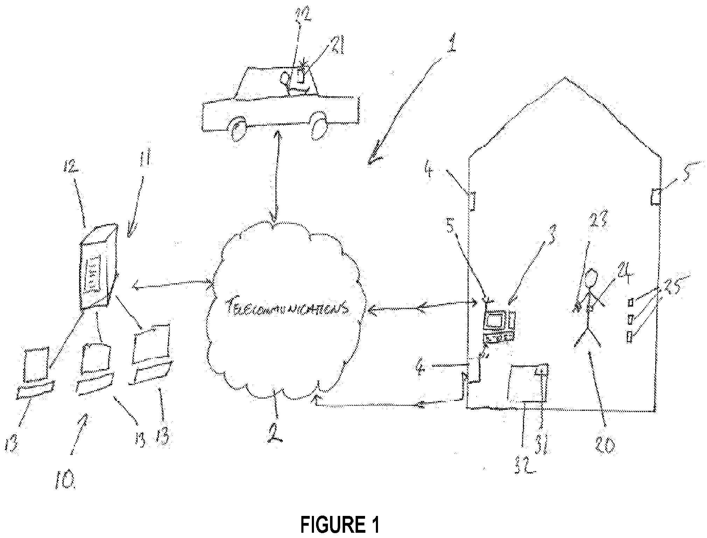

Referring to figure 1, a management system for facilitating the management of health and security, of a patient, generally designated by reference numeral 1, is illustrated.

In this embodiment, the management system, comprises a health and security management apparatus 3 which is arranged for use in the residence of a patient 20, and includes communications devices arranged for communications with a remote monitoring system 11, which also forms part of the management system 1. Communications device(s) of the management apparatus 3 are arranged to communicate health and security data with the remote monitoring system 11. In use, many management apparatus 3 may be placed in the residences of many patients, all the management apparatus communicating with the remote management system 11. The remote management system 11 acts as a central hub 5 from which the health and security of patients 20 with management apparatus 3 can be monitored. In this embodiment, the management apparatus 3 comprises a complete portal for communications with the patient 20. As well as health data and security data, in this embodiment, the management apparatus 3 is able to deal with systems in the home (home automation), and also provide a "social health" portal connecting the patient to their community, by phone, video and Internet. The communications apparatus 3 comprises a patient interface generated by a single application platform, providing a consistent interface which is easy to use and consistent across all facilities of the management apparatus.

In more detail, the management apparatus 3 provides a communications platform which includes a number of communications facilities, such as GSM 5, WiFi, Bluetooth™ (as will be discussed in more detail later). An ADSL modem 4 may be provided as a connection point to Internet or other telecommunications networks 2. Other communications facilities may also be included. One of the advantages of providing a plurality of communications facilities (e.g. mobile), is communications redundancy. If one of the facilities is not operating properly, one of the other facilities may be used (see later).

In operation, the management apparatus 3 is arranged to communicate via any form of communications available e.g. internet, landline telecommunications, POTS (plain old telephone system), mobile (GSM) or any other communications, with the monitoring system 11 at a remote location 10. In this embodiment, communications is preferably via a broadband cable connection (such as provided by the National Broadband Network in Australia) and by GSM. In other embodiments, however, communications connection may be by any available infrastructure.

At the remote location 10, the monitoring system 11 is provided to monitor communications and provide communications back to management apparatus 3. The monitoring system 11 may be arranged for communications with many such management apparatus 3 placed in many patient residences. The monitoring system therefore hosts health and security monitoring for many patients. The remote monitoring system 11 may comprise a computing arrangement of any known architecture, such as a server 12 and client terminals 13 via which health operatives can monitor the health and security of patients being tracked by the monitoring system 1.

In this embodiment, the management system 1 also comprises a medical professional 22 (such as a Nurse) device 21 which may be carried by a medical carer and which may be appropriately configured to receive and enter medical and administrative information regarding patients. In an embodiment, the medical professional device 21 may be an appropriately configured tablet computer or smart phone.

The medical carer 22 (who may be a community nurse, for example) may receive communications from the monitoring system 11 or from the management apparatus 3 via their device 21, to alert the medical carer 22 to actions that may need to be taken on behalf of the patient 20.

The management apparatus 3 is arranged to receive signals (by WiFi, Bluetooth™ or other local communications facilities) from health sensors 24, which may include heart rate monitors, blood pressure sensors and any other health sensors. The management apparatus obtains this health data and communicates to the monitoring system 11. Health data may also be input via a patient interface to the management apparatus 3, for transmission to the monitoring system, 11.

The management apparatus 3 is also arranged to receive data from security sensors 4,5, such as burglar alarms, smoke alarms and other security sensors. The management apparatus 3 is arranged to transmit security data onto the remote monitoring system 11.

The management apparatus 2 is also arranged to receive signals from personal alarms, such as a wearable personal alarm 23. The management apparatus 3 patient interface also comprises a patient alarm interface (e.g. an alarm button or touch pad or touch screen interface) where a patient can advise the monitoring apparatus 3 of an alarm and the alarm title will be transmitted to the remote monitoring system 11.

Both the health and security of the patient 20 can therefore be monitored remotely by the remote monitoring system 11.

In an embodiment, security information may be provided from the remote monitoring system 11 or from the security arrangement 3, 4, 5, to a security operative, who may have a device similar to the medical professionals device 21, but for receiving security related information (and also entering security related information). In another embodiment, the medical carer 22 may also deal with security and, if necessary, alert a security operative.

In this embodiment, the management apparatus 3 also comprises a home automation arrangement. The home automation arrangement may be implemented by appropriate software and hardware in the management apparatus. The home automation arrangement is configured to enable the patient 20 to operate devices in the home. The arrangement may provide signals to actuators 31 to operate devices such as washing machines, fridges, or other devices 32, remotely from the management apparatus 3. The home automation arrangement 30 may also, in an embodiment, be operated from the monitoring system 11. In an embodiment, a medical carer or other operative at the monitoring system 11 may remotely control devices in the patient's 20 residence, such as heating, air conditioning and other devices. Remote operation of the home automation arrangement may be particularly useful for infirm, or disabled patients, or patients suffering from early stage dementia.

The management system 1 of the present invention 1, including the monitoring system 11 and management apparatus 3, advantageously facilitate care in the patient residence. This avoids or at least delays the need to care for elderly, disabled or otherwise infirm patients in an institution such as a hospital.

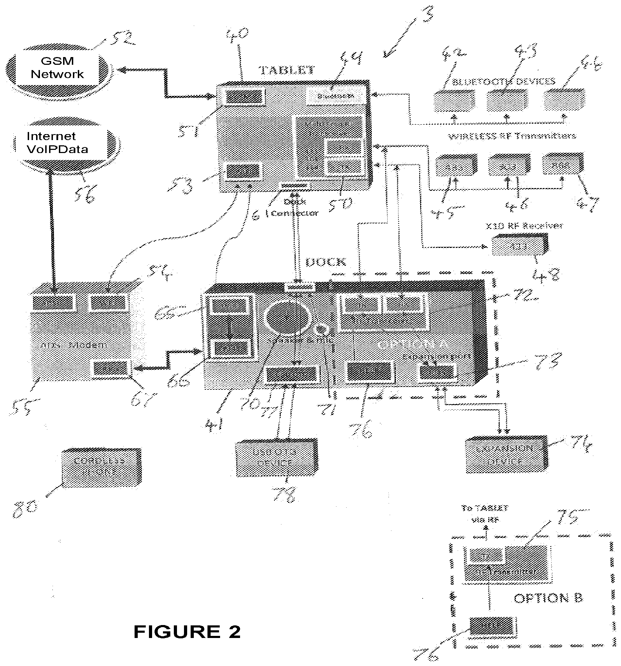

Figures 2 to 4 illustrate in more detail components of the management apparatus 3 which is arranged in the patient 20 residence. The management apparatus 3, comprises a portable unit 40 and a dock unit 41. The management apparatus 3 comprises an RF transceiver 72 and Bluetooth™ communications 49 for communication with devices in the patient's residence. In this embodiment, devices include Bluetooth™ devices 42, 43, 44 (there may have been more or less devices then shown). The Bluetooth™ devices may include various health and security sensors, arranged for use by the patient or placed around the residence. In addition, or alternatively, wireless transmitters 45, 46, 47 may be provided as sensor and security devices. Bluetooth™ and wireless devices may also be provided for home automation.

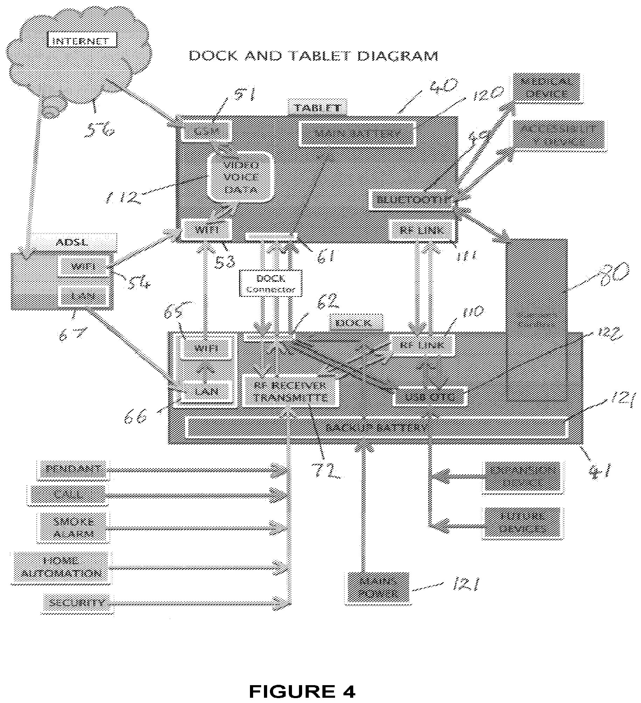

In the embodiment of Figure 2, the portable unit 40 comprises a Bluetooth™ receiver 49 and RF transceivers 50, for communications with the remote Bluetooth™ and wireless devices. A patient can receive information from these devices and control the devices via the portable unit 40. Figure 4 shows a slight variation on the embodiment of Figure 2. In Figure 4, the RF transceiver 72 is placed in the dock only and an RF link 110,111 allows communications received by the RF transceiver 72 to be transmitted to the portable unit 40. Transmissions can also be communicated to the portable unit 40 from the dock unit 41 when they are docked via dock connector 61, 62.

The portable unit 40 comprises circuitry enabling GSM communications 51 with a GSM network 52. It also incorporates WiFi circuitry 53 for WiFi communications. In this embodiment, WiFi communications 53 may enable communication with WiFi 54 in an ADSL modem 55 arranged to communicate with a telecommunications network such as the Internet 56, for VOIP and data for example. The mobile GSM network 52 may also allow communications via the Internet 56 (see Figure 4), as is known. It will be appreciated that allowing for ADSL and GSM communications allows redundancy of communications pathways. If the mobile network is not working, then the cable network may be utilised by ADSL (could be broadband or POTS or any available cable network), or vice versa. As the management apparatus can be considered to be an emergency critical system, redundancy of communications is a significant advantage. The portable unit 40 is configured for different communications facilities, being video, voice and data 112. If the network for some reason is not able to cope with data intensive communications, such as video, then voice may still be available. If not voice, then simple data communications (e.g. SMS) may still be available. This redundancy of communications facilities allows for variations in the quality of the networks, while still maintaining mission critical functions, such as the ability to provide an emergency alarm. In this embodiment, for emergency alarms and other critical communications, the management apparatus 3 is arranged to try each communications facility until the communication has been made.

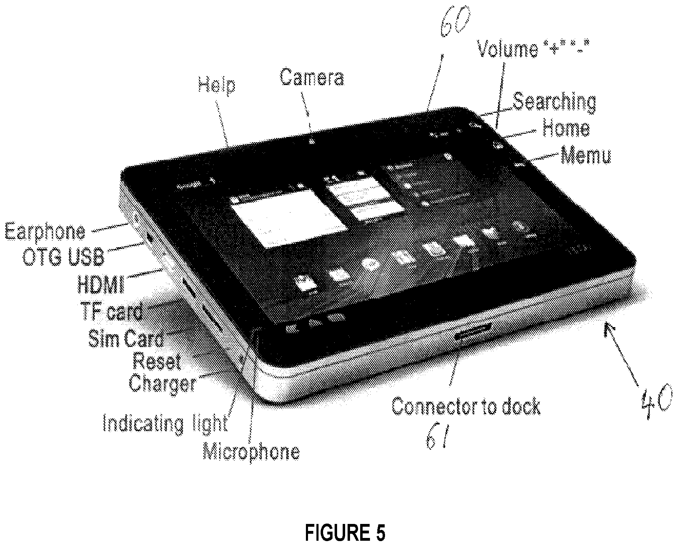

In this embodiment, the portable unit 40 comprises a tablet computing device configured for the health, security, home automation and other communications functions. It comprises a portable interface element in the form of a touch screen interface 60 (figure 5). This forms part of a patient interface of the management apparatus 3.

The portable unit 40 also comprises a dock connector 61 which is arranged to dock and connect with the dock unit 41, via connector point 62.

The portable unit 40 is arranged for portability, so that it can be carried around the home by the patient 20 and can also leave the home. The patient may take the portable unit 20 out with them so that the health and security functions are still available wherever they go. To facilitate portability of the portable unit 40, other devices providing functionality in the home can be kept in the dock unit 41. That is, facilities can be divided between the dock unit 41 and tablet 40, so as to ensure the tablet 40 remains portable, and still provides the functions needed for health and security of the patient when they are away from the home. The dock unit 41 may provide enhanced facilities which would not be suitable for portability.

The tablet 40 comprises a battery 120 for providing power to the tablet 40 when it is undocked from the dock unit 41. When docked, power is provided via the dock connector 61, 62 to the tablet 40, from mains power 121. The main battery 120 in the tablet 40 is recharged when the unit is docked.

The dock unit 41 is also provided with a relatively high capacity backup battery 121. In the case of failure of mains power, the backup battery provides charge to the main battery 120 as well as providing power to the management apparatus 3 when the tablet 40 is docked. The backup battery 121 is arranged to have a capacity to conform with emergency standards in various jurisdictions. In this embodiment, the backup battery has a capacity of 40 hours, which is greater than the standards requirement in Australia.

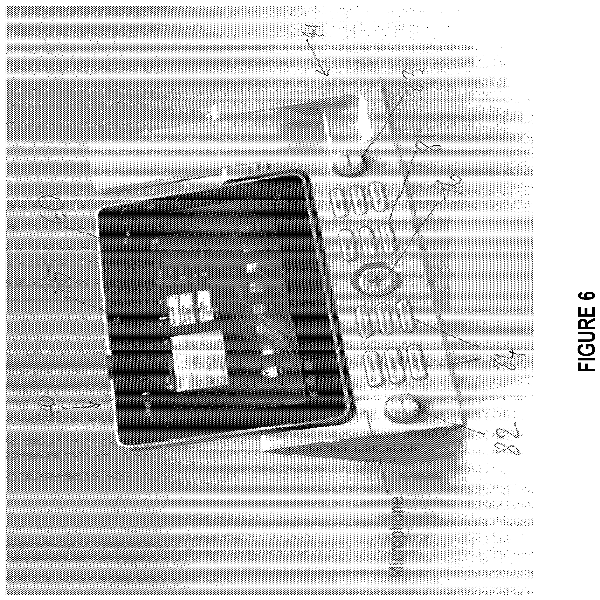

The dock unit 41 is arranged to dock the tablet 40 so that the tablet 40 is held in a relatively upright position (see Figure 6) for ease of viewing of the touch screen interface 60 by the patient 20. This is particularly convenient for old, infirm and disabled people.

The dock unit 41 has a WiFi transceiver 65. The WiFi transceiver 65 may communicate via an RJ 45 socket 66 which co-operates with a corresponding RJ 45 connector 67 in the ADSL modem 55. The tablet 4 may therefore communicate with ADSL modem and remote system directly via WiFi 53 and 54 or via the dock WiFi 65 and RJ 45 connections to the ADSL modem. The connection to the Internet 56 allows communications with the Internet generally, not only the remote system 11. Similarly the connection via the GSM network 52 allows communications anywhere. The monitoring apparatus 3 can therefore be used to communicate data, voice, video with any system not only the remote system 11. The system is programmed, however, to communicate health data and security data directly to the system 11 (without the patient 20 having to make the connection. For example, the connection may always be open "on").

It will be appreciated that although particular types of communications facilities are implemented in this embodiment, the invention may utilise other types of communications facilities, without limitation.

The dock unit 41 comprises audio facilities, in the form of a speaker and microphone 71 which are of enhanced quality as compared with the corresponding speakers and microphone facilities in the tablet 40. The dock therefore provides an enhanced audio interface. When docked, the tablet 40 can be used for remote communications via the interface 60, which can enable video transmissions, utilising the enhanced audio facilities of the dock 70, 71. This may be particularly useful for patients who have difficulty hearing and/or speaking.

In this embodiment, the dock unit 41 also includes radio transceivers 72. These can communicate with the tablet and also with the wireless devices 45, 46, 47, 48. The dock can therefore be used for control of various wireless devices and also to receive information, from wireless devices and communicate with the radio transceiver 50 of the portable unit 40 or via RF link 110.

In this embodiment, an RJ 45 expansion port 73, or USB OTG 122 (Figure 4) may allow addition of expansion devices 74. The expansion devices 74 may be any device for any type of functionality. In an embodiment, the expansion device 74 is a home automation control device with can be used to control wireless transmitters/receivers and Bluetooth™ devices to control home automation.

In an alternative embodiment, a transceiver 72 is not included and only a wireless transmitter 75 to transmit to the tablet 40 ("Option B", Figure 2).

An alarm button 76 ("HELP") is provided on the dock 41. If this is activated by the patient, it provides an alarm via the tablet 40 to the remote monitoring system 11 (via GSM and/or ADSL). A medical operative is therefore able to respond. A "soft" alarm button may also be provided by the screen interface 60 of the portable unit 40. In addition, a "hard" button may be provided on the portable unit 40 for providing an alarm, in a similar way to the hard button 76 on the dock unit 41. This ensures that a patient will always be able to call for "help". In addition an external device such as a pendant, may be worn by the patient and communicate with the management apparatus 3 to send an alarm.

The monitoring system 3 also includes a cordless hand phone 80, which may communicate with the tablet or dock via Bluetooth™, WiFi, or any other communications medium, for enabling communications externally (GSM or VOIP), to the remote system 11 or any other communications.

The sensor devices may comprise any available health parameter sensor or device. These may include any device for collecting clinical measurement such as blood pressure, BSL, ECG, INR, SPO2, temperature, urine, weight and respiratory rate and any other sensor. The sensors may automatically communicate with the tablet/dock. Alternatively, the patient 20 may take readings and input them to the tablet/dock via the patient interface. The management apparatus 3 may be arranged to deal with currently available health parameter devices.

Clinical measurements can be faxed, emailed or otherwise communicated to the remote location 11 or medical carer 22.

In this embodiment, a two way messaging system between the remote location 11 and the tablet 60 is also implemented in the form of a "widget". This can always be open so that communication is always available. The Widget may be used to communicate a reminder to the patient 20 that they need to take medication, for example. It can be used to communicate any information. An example of operation of the widget is given later on in this description. The tablet also includes a camera 85, which facilitates video communications with remote location (or any other location that is video communication enabled).

In an embodiment (not the embodiment shown) the tablet 40 may include GPS functionality. This enables the location of the tablet to be tracked. For example tablet 40 location may be tracked from the remote monitoring system 11, so that a medical carer is always aware of where the tablet 40 is. If the patient leaves the home with the tablet 40 therefore, and they provide an alarm, their location may be tracked via GPS.

Any GPS and GSM functionality may be turned off when the tablet 40 is docked, in order to save on power and also avoid interference.

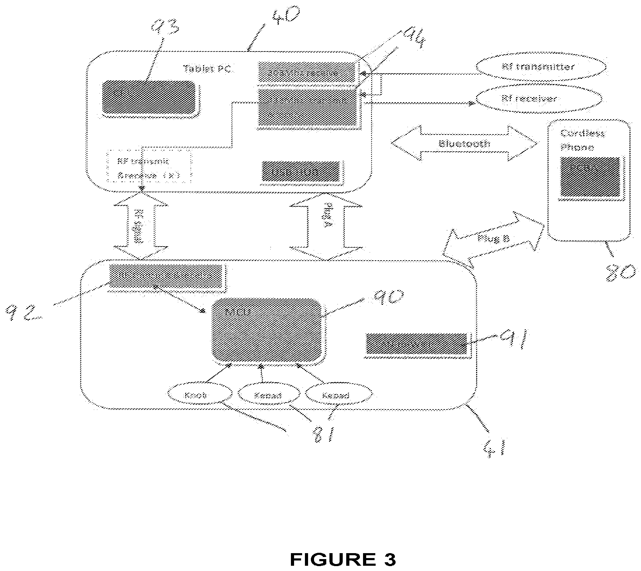

Figure 3 is another schematic diagram of the dock unit 41 and portable unit 40 and phone 80, showing further components.

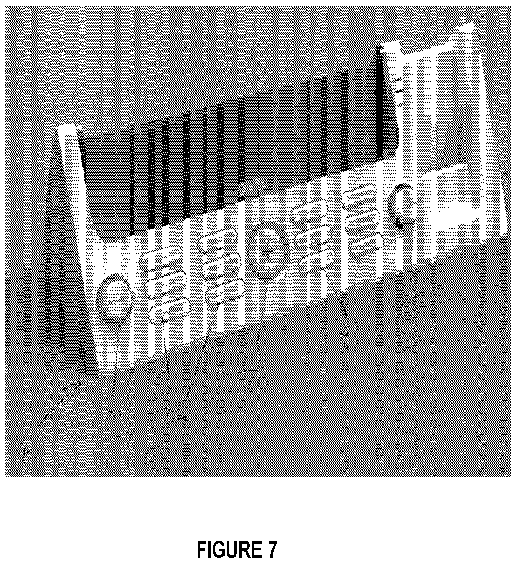

The dock unit 41 is provided with an interface comprising a keypad 81 (also see figure 7), the keypad 81 includes rotatable knobs 82, 83, in this embodiment being for the adjustment of brightness and volume of the screen on the portable unit 40. The keypad 81 also includes a number of relatively large push buttons 84 (figure 7) which can be used to control various functions of the monitoring apparatus 3. In this embodiment, the function of the push buttons 84 may be configured by the patient or by the medical professional. Having these relatively large push buttons 84 for operation of the monitoring apparatus 3 may be particularly useful for old, infirm or disabled people that are not comfortable with the use of a tablet touch screen for input/control of the monitoring apparatus 3.

Referring to Figures 6 and 7, there is a single HELP button 76 on the dock which is used to activate the medical alarm. There are four buttons on the bottom row that are not programmable. First on the left is the HOME button which will return the user to the HOME screen of the tablet. Second from left is NOTIFICATION, which will display the current notifications on the tablet (see later). Third from left is CALL CENTRE, which initiates a voice or video call to the remote monitoring system 11. Fourth from left is SELF CARE, which will bring up a screen to display options for self medical care (see later). The second and third row up are programmable buttons, which can be used for speed dials, shortcuts to applications on the tablet and are programmed either by the tablet software, or could be reconfigured from the remote monitoring system 11.

An advantage of the monitoring apparatus 3 is the flexibility of the interface. A user not comfortable with computer technology may use the keypad 81 of the dock unit 41 and still obtain required functionality of the monitoring apparatus. Persons more comfortable with technology can use all facilities provided by the sophisticated touch screen interface on the tablet 40. In this embodiment, as well as facilitating communications between the remote system 11, medical professional's device 21 and monitoring apparatus 3, for the provision of health information and alarm information, the monitoring apparatus provides all the functionality available for a typical tablet computer. For example, "apps" may be downloaded for different functions. People who are more comfortable with computing technology thus have the full range of functionality available to them.

In addition, as discussed above, the monitoring apparatus 3 can be used to control devices in the home (home automation). As well as the monitoring apparatus 3 being able to be used by the patient 20 to control home automation. Home automation may be controlled remotely by the system 11, a medical professional may therefore be able to control devices in the house, such as heating, air conditioning and other systems.

Referring again to figure 3, the dock unit 41 comprises a micro-computing unit (MCU) 90 for control of the dock unit 41. The MCU 90 may include memory and a processor. The dock and devices attached to it may communicate via WiFi 91 and RF 92 as referred to above, with other devices and the tablet. The tablet 40 has a CPU 93 and also communications devices as discussed above for radio frequency transmission 94, WiFi and Bluetooth™.

Transmitters and receivers in the tablet and operation of the touch screen, require a large amount of power. In use, the tablet 40 will generally be docked in the dock 41. The dock 41 comprises a back-up power module (to meet jurisdictional standards of personal alarm systems). Connecting with dock unit 41 enables recharging of the tablet 40 battery. The dock unit may be connected to the mains (so its battery is always charged).

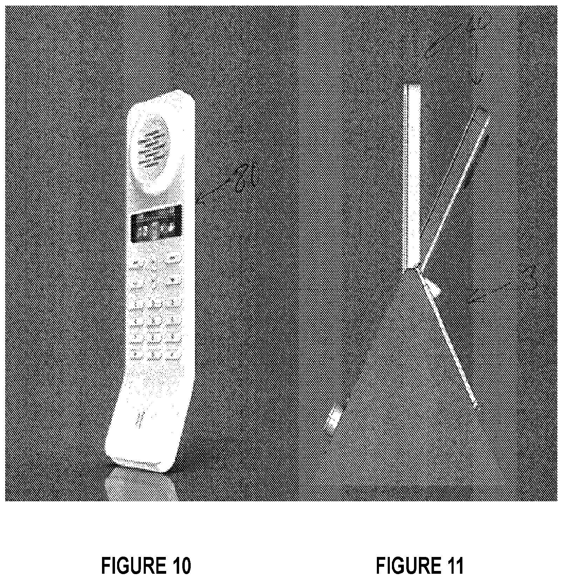

As discussed above, the dock 41 holds the tablet 40 in the upright position for ease of use by the patient 20. The angle of the tablet 41 may be adjusted, in this embodiment between 0 and 30 degrees, as illustrated in figure 10.

The management apparatus 3 effectively operates as a "portal" to provide services to facilitate health and security care of a patient while the patient remains in their residence. The management arrangement allows for tele health services to be provided via the management system 1. Clinical information may be provided to the remote system and to doctors. Video calls may be made to discuss health.

The management apparatus 3 may also be used to enable medical services such as script filling. For example, a virtual link may be provided with the doctor who will review the vital signs and write a script. The script could be then sent electronically to the patient and/or pharmacy. The medicines filling out the script could be provided separately (e.g. by post or courier).

The system also provides the functionality of a personal response system, allowing for medical alarms to be transmitted remotely and home security to be monitored.

The system also facilitates home automation. Systems in the home can be controlled remotely or locally using the management apparatus.

Further, the management arrangement promotes social health, by keeping the patient connected to the community via the portal. This can be via the Internet, telephone or other communications facility. In this embodiment, the remote monitoring system is arranged to provide community services. For example, games such as "bingo" games, can be transmitted across the network to the management apparatus. Educational information can also be provided. Further, an Internet connection to social networks, such as Facebook, is facilitated.

In an embodiment, the remote monitoring system enables a portal whereby community services can be provided to the management devices for a variety of patients. For example, the administration of a community village (e.g. a retirement village) may be allowed access via the portal to broadcast information about village services, village functions and any other information. Access may be by way of the village administrator's computing system and a web enabled portal served by the monitoring system 11.

Further, other services, such as Internet tv can be provided via the management apparatus 3. In this embodiment, music and tv can be streamed wirelessly to HiFi and flatscreen tvs in the home.

The management apparatus can therefore provide a full service portal, providing all the above services, in a single interface.

The following are examples of operation of the monitoring system 1 in accordance with this embodiment.

### Example 1

A patient managing their care at home uses the management apparatus 3 to record clinical measurements, which can then be monitored by a health professional via the remote monitoring system 11, and reported to the patient's doctor, as required. The patient 20 has access to clinical measuring devices 24, 25 which may operate via wireless or Bluetooth™, or which may be manually operated and the patient then uses the management apparatus 3 to enter the measurement.

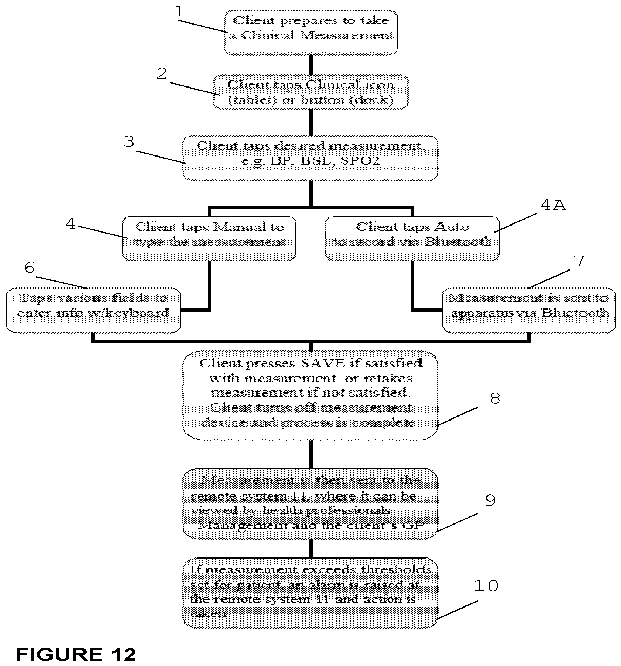

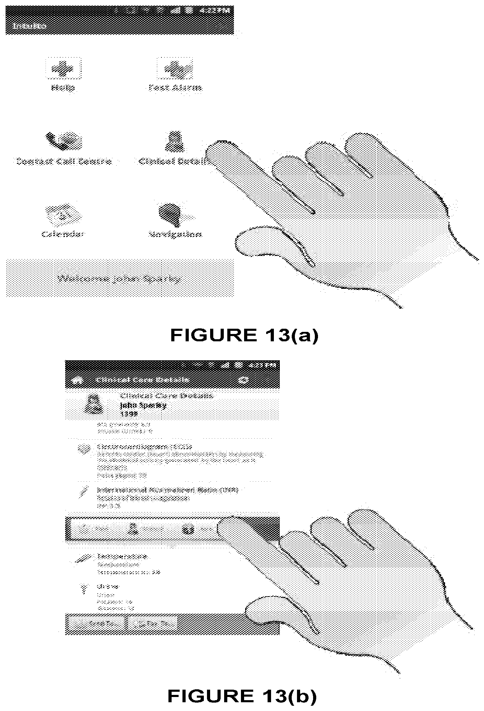

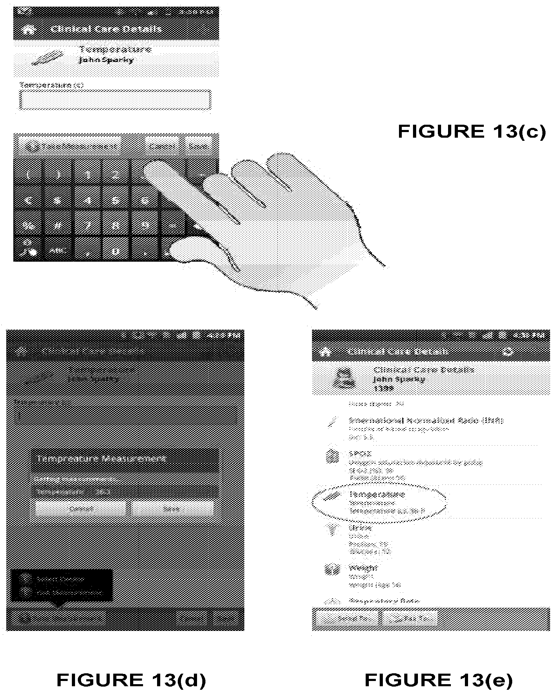

Referring to figure 12:
1. A patient needs to take a clinical measurement (e.g. Blood Pressure, Blood Glucose Level, Pulse, SPO2, etc.)
2. Patient taps the Clinical Details icon (on screen 60) or button 84, (on the dock 41) to access the clinical functions (Fig 13(a)).
3. Patient taps the appropriate measurement they wish to take, e.g. Blood Pressure.
4. Patient taps "Manual" if they wish to manually enter the measurements, or "Auto" if they wish to collect them via Bluetooth™ (Fig 13(b)).
5. Patient then starts the measuring device and takes the measurement.
6. If entering measurements manually, patient taps on the various fields on screen and enters the measurement via the onscreen keyboard (Fig 13(c)).
7. If entering automatically via Bluetooth™, patient waits a moment until the measurement is sent to the management apparatus 3 and automatically displays.
8. If satisfied the measurement was entered properly, patient presses SAVE to record the measurement in their electronic health record (Fig 13(e)).
9. The measurement is sent to the remote monitoring system 11 where it can be viewed by health professionals, management and sent to the client's doctor, or other medical professionals.
10. If the measurement exceeds the threshold set for the patient, an alarm is raised at the remote monitoring system 11 and action is taken. For example, a qualified nurse may contact the patient by phone or video call (via the management apparatus 3) and determine whether assistance is required. The nurse provides whatever assistance may be required, which could include calling a contact person, notifying the patient's doctor or even calling an ambulance.

The client's doctor is informed of the measurements. This can occur in various ways:
a) Fax or email the measurements periodically directly from the monitoring apparatus 3.
b) The health professional at the remote monitoring system 11 can send a printed report of the client's measurements, either by a fax, or e-mail or post.
c) The doctor may wish to view the measurements at any time. In that case, they can log into the remote monitoring system 11 and view measurements for their patients at any time.

### Example 2

The patient can use the management apparatus 3in order to summon assistance from the remote monitoring system 11 in the case of an emergency. The patient has a transmitter 23 which is worn wristwatch style (or could be worn as a pendant, or even wall-mounted or mounted at some other part of the residence). The patient may also have other devices in their residence which are able to transmit alarms, such as smoke detectors, burglar alarms 4, 5, and other alarm devices.

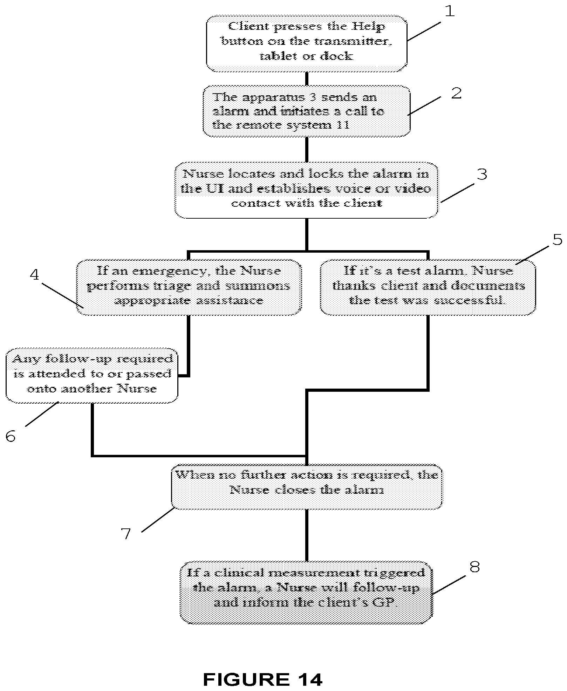

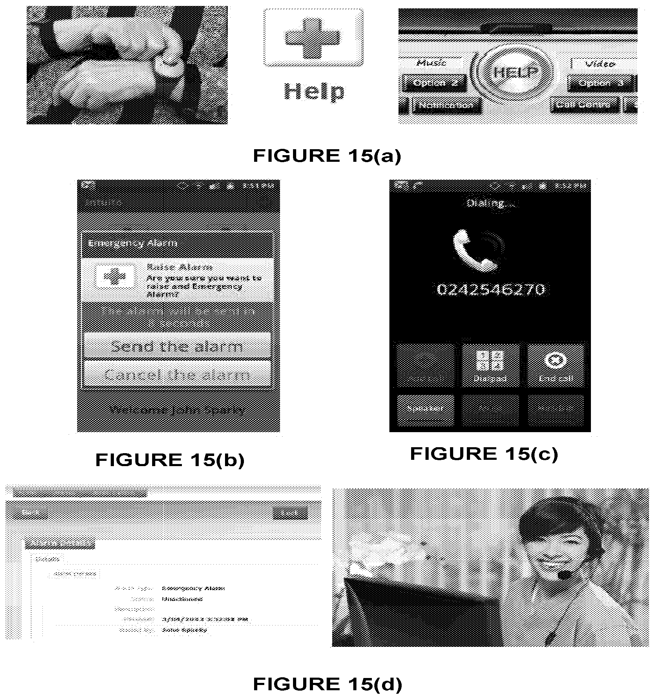

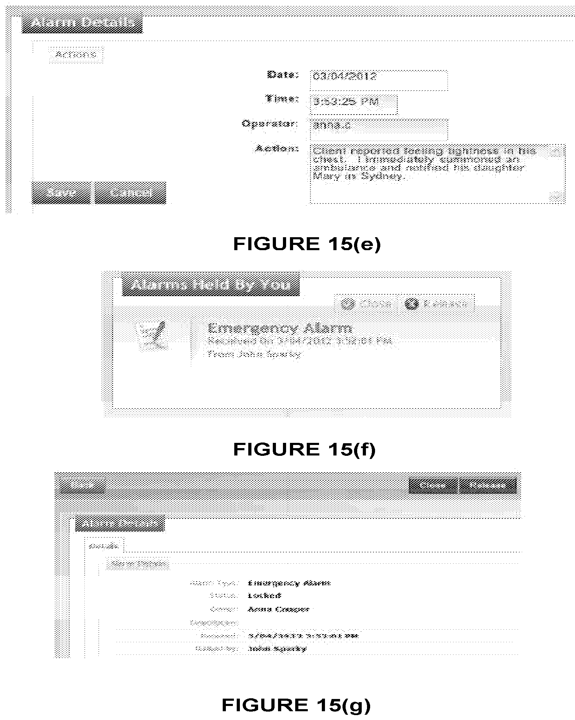

Referring to Figure 14:
1. Patient presses the HELP button 76, on their transmitter 23, tablet 40 or dock 41, Figure 15(a).
2. The management apparatus 3 sends an alarm and initiates a call to the remote monitoring system 11 where a Nurse will establish voice or video contact with the client and determine whether it is an emergency alarm or a test (Figures 15(b), 15(c).
3. The Nurse locates the corresponding alarm in the Remote System 11 User Interface (UI) and locks it to identify it is being actioned (Figure 145(d)).
4. If an emergency, the Nurse initiates a triage process and takes any necessary action, which could include notifying the patient's nominated contacts, summoning emergency services, or calling a neighbour. Any actions taken are documented in the UI (Figure 15(e), (f), (g)).
5. If non-emergency assistance or maintenance is required, operator contacts appropriate party and advises client (for maintenance issues, operator may need to connect client with help desk), or if it is a test alarm, then Nurse thanks the client and documents the test for success.
6. If further follow-up is required, operator can place alarm on hold for a period of time, or release it for another Nurse follow-up.
7. When no further action is required, Nurse closes the alarm.
8. The alarm may have been triggered by recording a clinical measurement that has exceeded predetermined thresholds.
   a) Based on the medical information available the Nurse may initiate a video conference.
   b) The nurse provides whatever assistance is required, which could mean calling a nominated contact, notifying their doctor, or even calling an ambulance (reference numeral 8 in Fig 14).
   c) The client's GP or Specialist is informed of the measurement.

The alarm may have been triggered by another device capable of transmitting an alarm, e.g. a smoke detector.
a) The nurse will attempt to establish voice or video contact with the client.
b) The Nurse will summon Emergency Services as appropriate.

### Example 3

A medical carer, such as a Nurse, can use the monitoring system 1 to initiate a video conference rather than providing a face to face visit (e.g. for medication prompt, welfare check, wound consult or case conferencing).

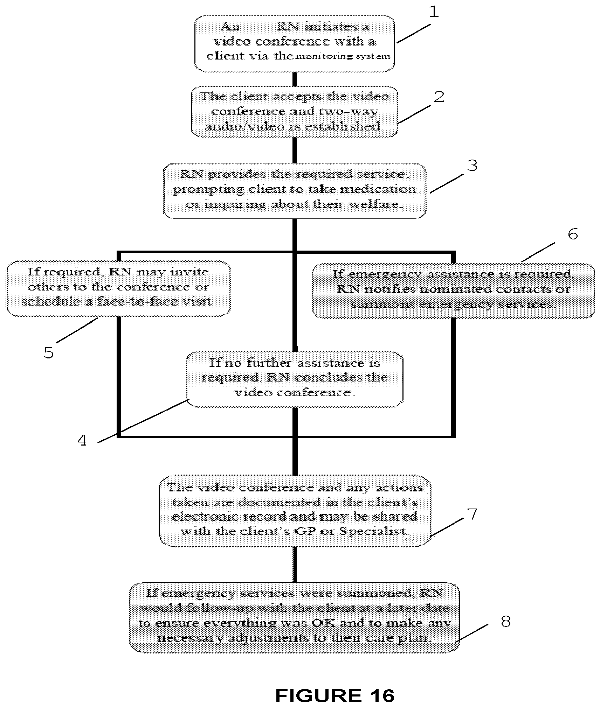

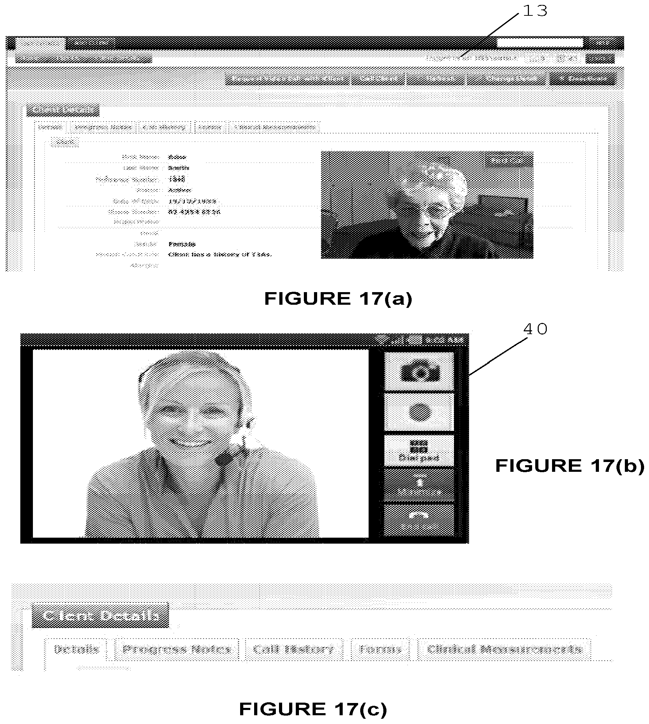

Referring to Figure 16:
1. The nurse initiates a video conference with the client via the management apparatus 3 (tablet 40). Figure 17(a) illustrates a representation of what the nurse might see on their monitor 13 and Figure 17(b) is an illustration of what the patient might see on their tablet 40. In step 2 the client has accepted the video conference. Two way audio/video is established. The dock unit 41 supports the tablet 40 in a conveniently upright position for the patient to comfortably attend the video conference.
2. The nurse provides the required service, prompting the client to take their medication or enquiring about their welfare, as appropriate.
3. The nurse may feel confident that the video conference was sufficient and they conclude the video conference.
4. If the nurse feels it's appropriate they may invite others to the conference or schedule a face to face visit.
5. If there is an emergency, the nurse takes immediate action. This could include notifying the patient's nominated contacts, summoning emergency services, or calling a neighbour to assist. The patient's GP or Specialist may also be informed, if appropriate. The nurse will follow up with the patient at a later date to ensure everything was alright or to adjust their care plan if necessary (reference numeral 8).
6. The video conference and any actions taken are documented in the client's electronic record (Figure 17(c)) on the medical carer administrative system, and may be shared with the client's GP or specialist.
7. If emergency services were summoned, the Nurse will follow up with the client at a later date to ensure everything is okay and to make any necessary adjustments to their care plan.

### Example 4

A medical professional 22, such as a Nurse, uses the medical professional device 21 and the medical carer administrative system hosted by remote system 11 for service provision as they record their attend times, kilometres travelled and record progress notes, forms and clinical measurements of the patients they attend to. The medical professional may be a community nurse, for example, provided with a medical professional device 21, such as a smart phone or tablet which has been appropriately configured with the medical carer administrative system, for interfacing and access to telecommunications, the remote monitoring system 11 and the management apparatus 3 of the management system 1. The nurse 22 may also have equipment able to take various clinical measurements when they attend a patient. If the equipment is Bluetooth™ capable it can communicate with the management apparatus 3 and monitoring system 1.

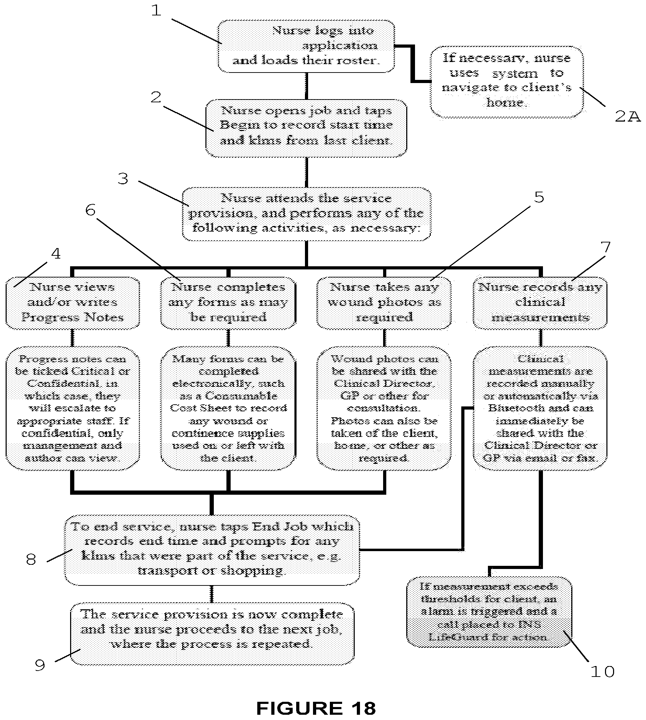

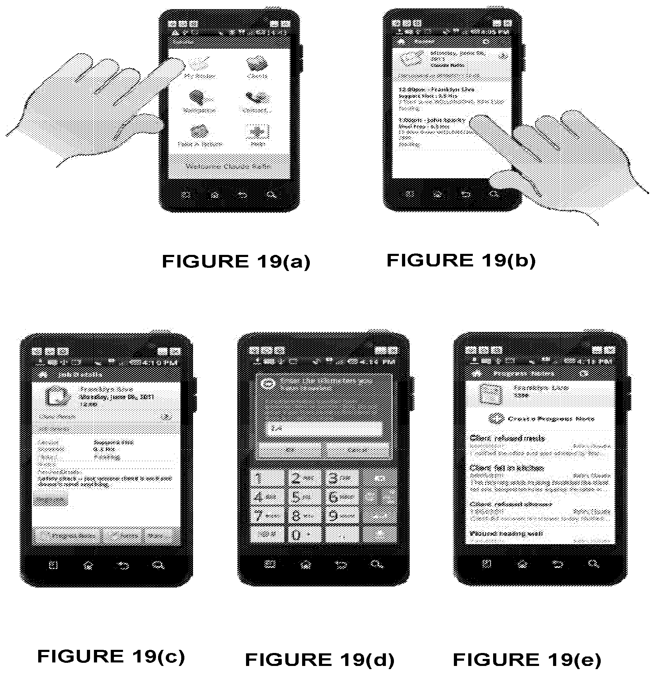

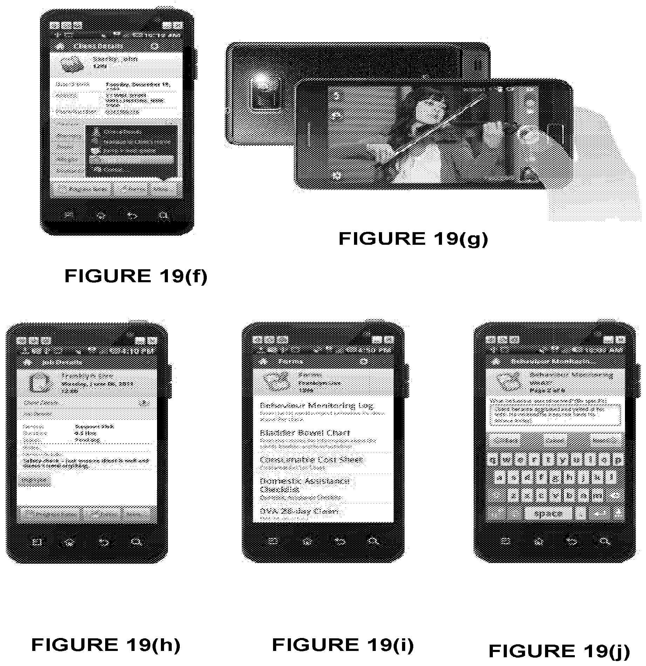

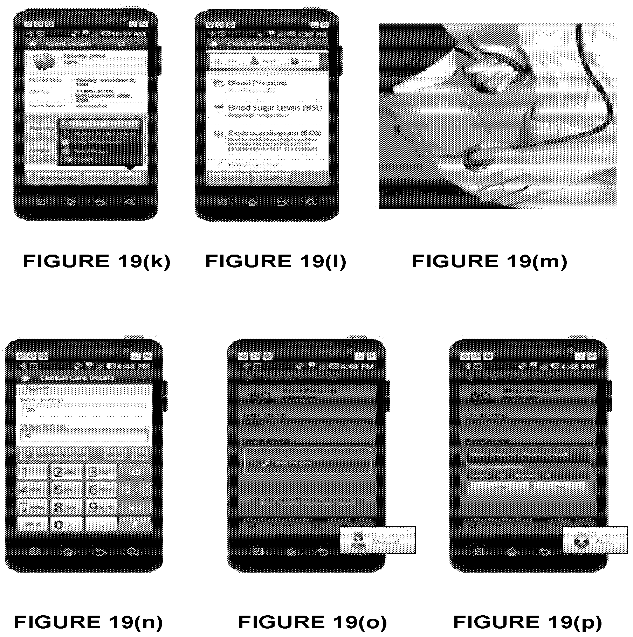

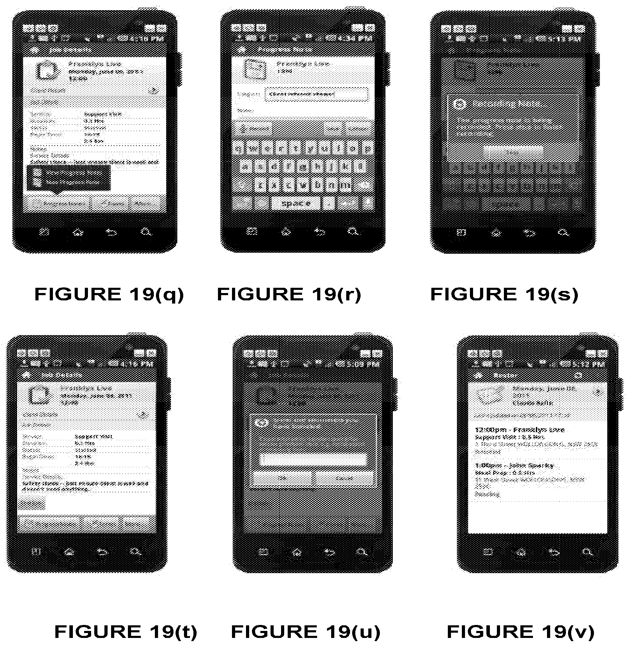

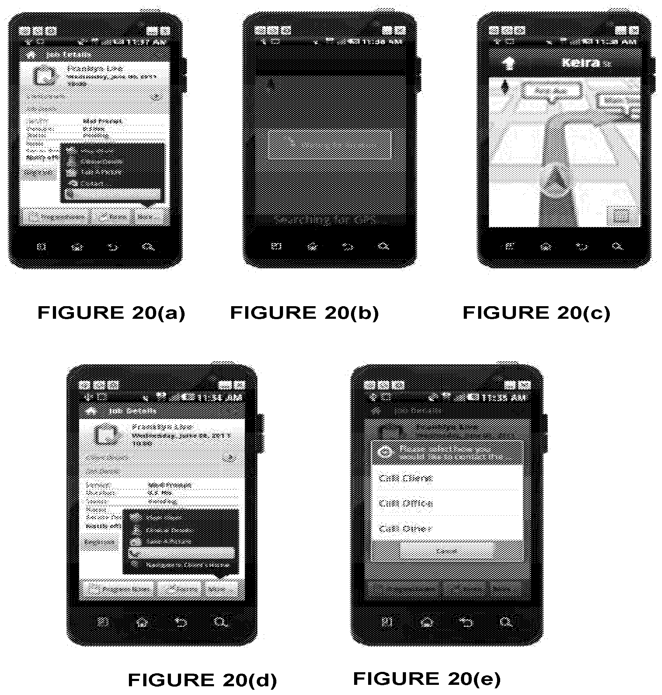

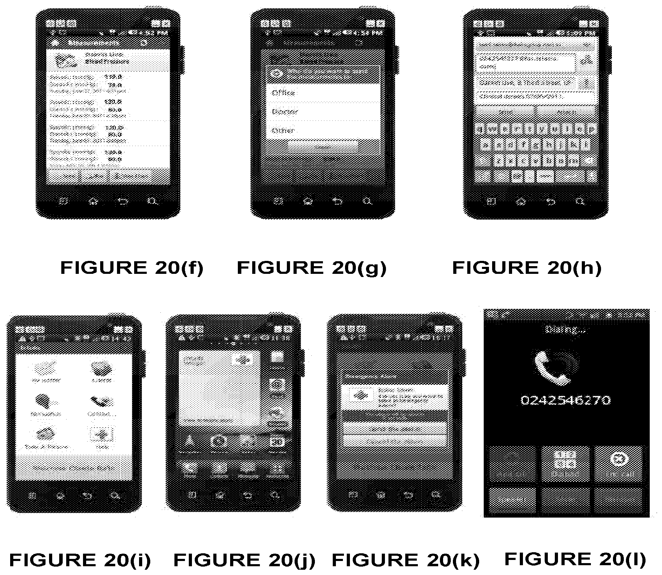

Referring to Figure 18:
1. Nurse logs into medical carer application on their smart phone 21, or tablet 21 and presses 'My Roster' to load their roster (Fig 19(a)).
2. The nurse taps the first job on their roster that they wish to attend (Fig 19(b)). This opens the job to reveal the service details (Fig 19(c). From this screen they can also choose to navigate to the client's home 2A, or view the client's details, including medical conditions and allergies.
3. The nurse attends the service provision, which will involve going to the client's home (they can use the navigation system provided by the nurse administrative system (see later description and Figure 20).
   The nurse taps 'Begin Job' to record the start of their attendance time and enters their kilometres travelled from the previous client (Fig 19(a)).
4. The nurse taps Progress Notes icon to view any notes entered since they last visited, or the 10 most recent notes if it's a new client, Fig 19(e).
5. In the event that wound care is part of the service provision, the nurse takes a photo of the wound which is stored with the client's electronic record (Fig 19(f), 19(g)).
6. The nurse completes any forms required during the service provision by tapping the 'Forms' icon and choosing a form from the list. Forms are completed using tick boxes, drop down lists, and typing (Figs 19(a), (i), (j)).
7. The nurse enters clinical measurements, if required, by tapping the 'More' icon and choosing Clinical Details, then choosing the appropriate measurement they wish to take, e.g. Blood Pressure.
8. To end the service provision, the nurse taps the End Job icon, at which time they are prompted to record any kilometres that were part of the service_provision (e.g. transport or shopping) Fig 19(t) 19(v).
9. The service provision is now complete and the nurse proceeds to their next job, where the process is repeated.
10. Measurements taken exceed thresholds set of the client and are thus a cause for concern.
   a) An alarm is raised in the INS LifeGuard Call Centre, and the alarm is actioned by a triage Nurse.
   b) The triage nurse provides whatever assistance is required, which could mean notifying the client's doctor, or calling an ambulance.

The nurse notices a wound or other such issue and wishes to consult with another party, e.g. the Clinical Director or GP.
a) The wound photo taken can be immediately sent to the consulting party via email for consultation
b) The clinical measurements taken can be emailed or faxed to the consulting party for review
c) The nurse documents the results of the consultation in the client's Progress Notes

Photos may be needed of the client, the home, or other - not just wounds. Intuito™ flags photos in the correct category.

Referring to Figure 20, there are illustrated some other functions that may be provided by the medical professional device 21 for the medical professional 22. Figures 20(a) to 20(c) show how the system provides GPS navigation to client's premises prior to commencement of service.

Figures 20(d) and 20(e) illustrate how the medical carer may contact the client or the remote management system 11 without leaving the service.

Figures 20(f) to 20(h) show how a doctor or the office (remote system) may be faxed regarding client parameters such as clinical measurements.

Figure 20(i) to 20(l) show that a HELP button may be used for any situation in which immediate/emergency assistance is required, whether for the client (perhaps an ambulance is required or the nurse (e.g. car broke down late at night). The HELP is available on every screen of the mobile application, as well as on the home screen of the phone itself, so assistance can always be summoned quickly. The resulting alarm may be handled by the remote monitoring system 11 exactly the same if it were an alarm from the client (see Example 2).

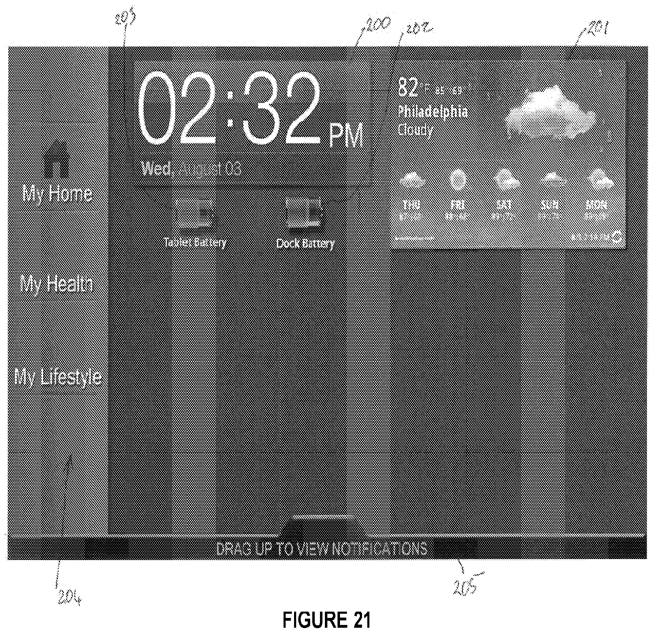

Figures 21 through 24 show some sample patient interfaces that may be generated by the management apparatus 3, in this case on the touch screen 60 of the pad unit 40. Figure 21 shows a "home screen". The date and time 200 is displayed on the screen and also the weather 201. It will be appreciated other information could be displayed. The charge status of the dock battery 202 and the tablet battery 203 are displayed on the screen. A menu 204 is provided which enables a patient to select other screens. At the bottom of the screen a tab "drag out to view notifications" 205 is displayed.

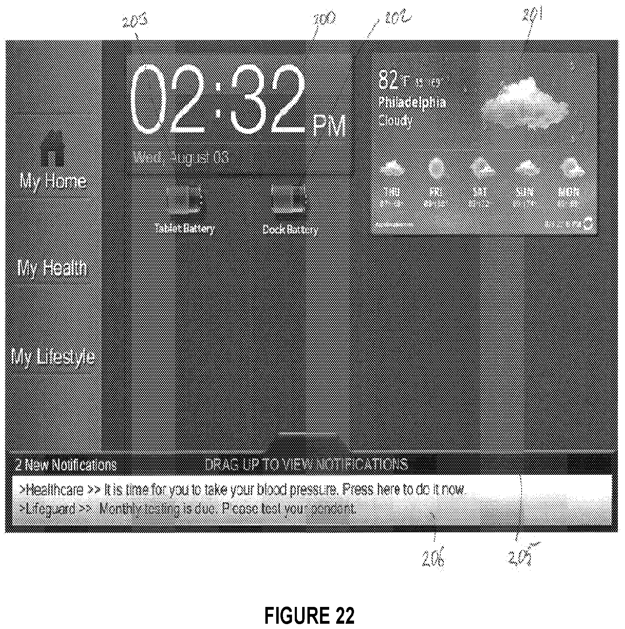

If the tab 205 is dragged upwards, notifications 206 (Figure 22) may be displayed. These notifications may be provided from the remote monitoring system 11.

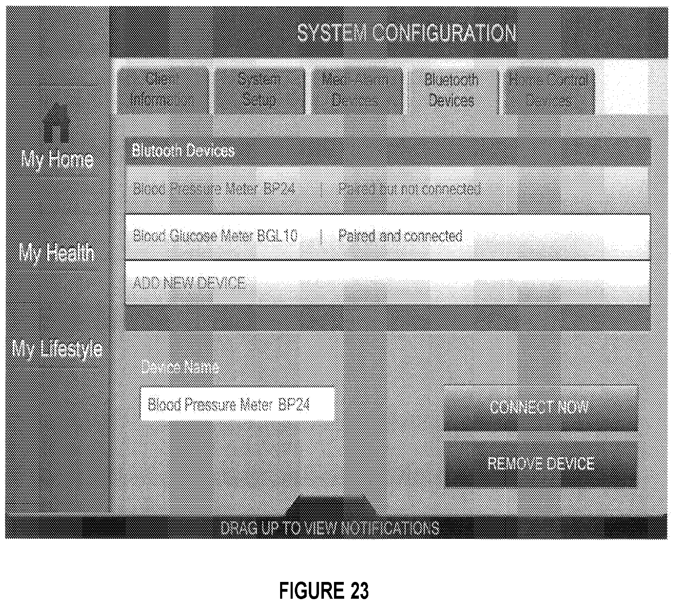

Figure 23 shows a "system configuration" menu. The menu displayed is "Bluetooth™ devices" that enables addition and subtraction of devices which can be connected via Bluetooth™ to the management apparatus 3.

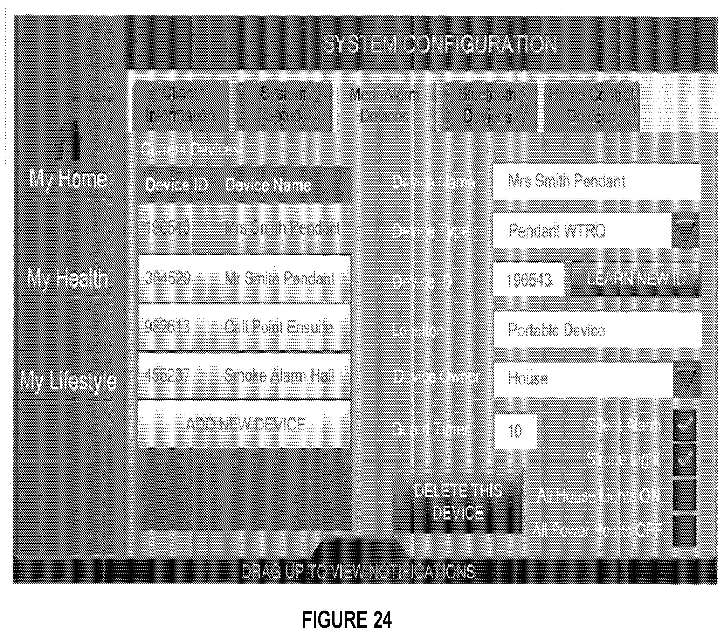

Figure 24 shows system configuration of various security alarm devices that can be added or taken away.

It will be appreciated that other screen shots may be provided in accordance with required functionality of the device.

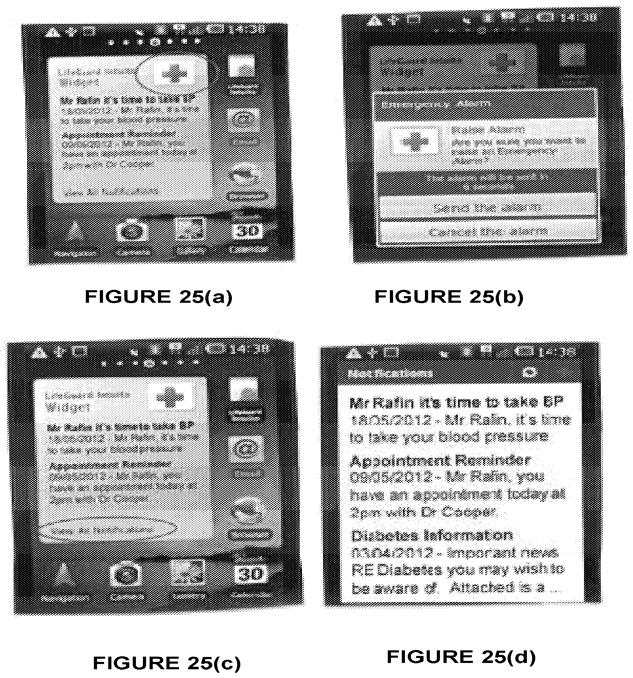

Figures 25(a) to 25(d) show various displays that may be provided by a widget which is permanently on, providing a permanently on messaging service with the management apparatus 3 and remote monitoring system 11. Figure 25(a) shows various notifications being provided by the widget. If the patient actuates the alarm (red cross symbol) then the screen shot shown in Figure 25(b) is generated asking whether the alarm should be sent.

Referring to Figure 25(c), if the patient selects "view all notifications", then the notifications are displayed via the widget (Figure 25(d)).

The widget may communicate any information.

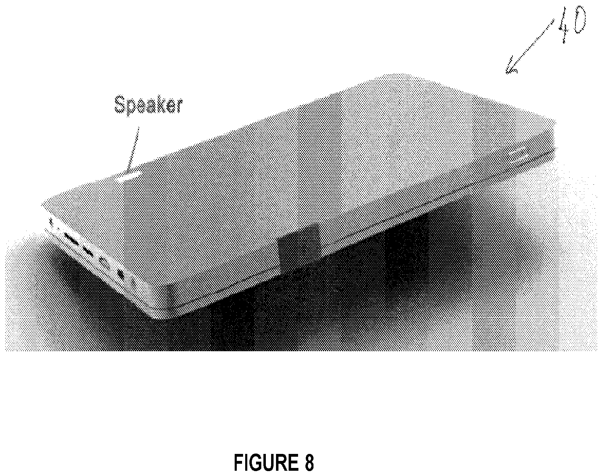

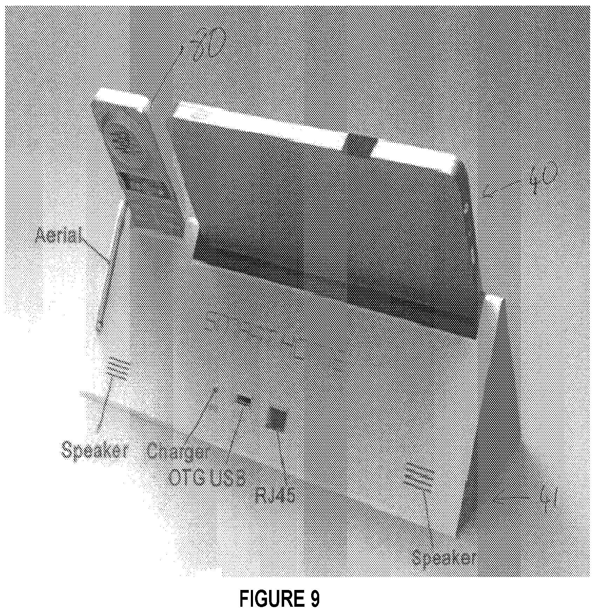

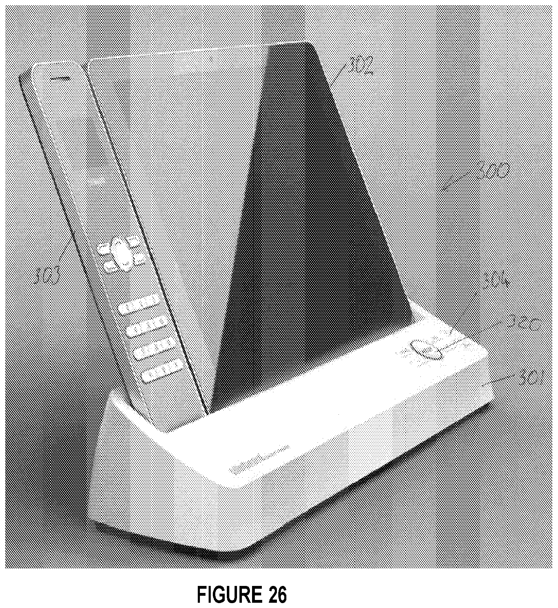

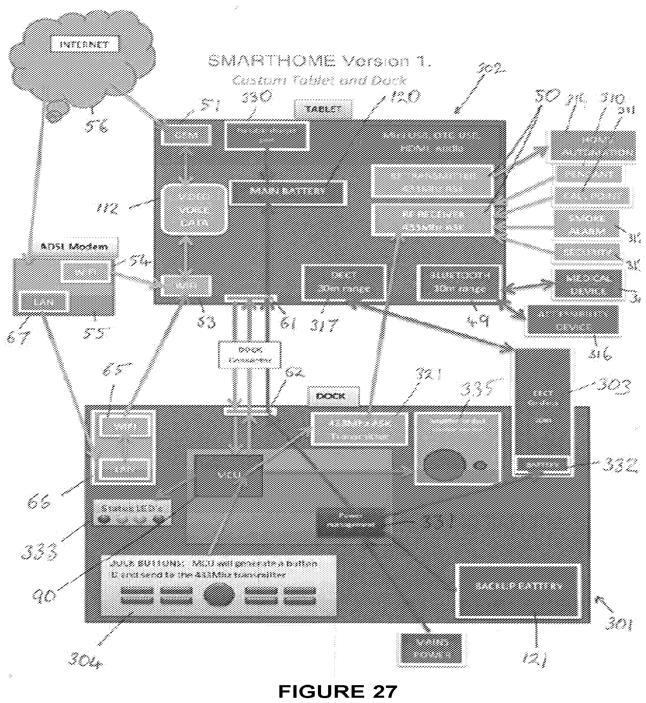

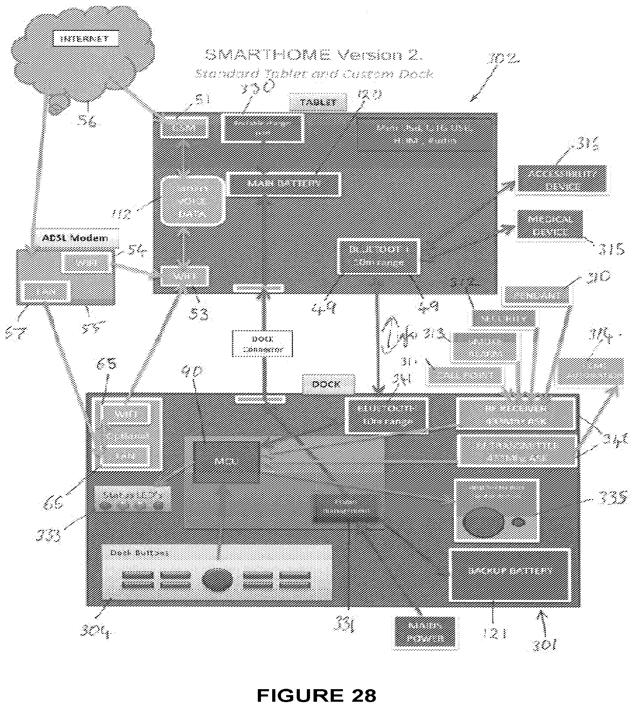

Figure 26 illustrates a further embodiment of a management apparatus in accordance with the present invention. This has similar features to the apparatus of the embodiment of Figures 5-11, but with some modifications in design and functional components (see description below in relation to Figures 27 and 28).

This embodiment of the management apparatus 300 also comprises a dock 301, tablet 302 and telephone handset 303 which have similar functionality to the equivalent components of the embodiments described above. The dock 301 includes hard buttons 304 on the right hand side of the front surface of the dock 301. The tablet 302 is separable from the dock 301 to be portable. As is the handset 303. The management apparatus 300 is compatible with the system described above in relation to Figure 1, including being compatible with the remote monitoring system 11 and medical professional device 21. The management apparatus 300 operates to provide substantially the same functionality as above embodiments.

Figure 27 shows one possible architecture for the management apparatus 300 of Figure 26. In Figure 27, the same reference numerals have been allocated to components which have the same functionality of the like-referenced components in Figures 2, 3 and 4. No further detailed description will be given of these components.

In the Figure 27 architecture, the tablet 302 incorporates the majority of the functionality of the management apparatus 300. It includes GSM 51 and WiFi 53 communications to allow remote communications, as discussed above in relation to the earlier figures. Video, voice and data is therefore enabled with remote locations, and the monitoring system 11.

The tablet 302 also includes a portable charger port 330, for charging the battery 120 of the tablet, when the tablet is not connected to the dock 301.

The tablet 302 has RF transmitter and RF receiver 50. These are arranged to receive signals from all security devices, such as PERS devices (e.g. pendants 301 and call points 311). The security devices may also be standard home security devices such as smoke alarms 312 and standard security alarms, door alarms, etc. 313. Any alarms received may be processed by the tablet (MCU not shown but present) and transmissions then made to remote systems (e.g. monitoring system 11 via GSM 51 or ADSL or any other communications mechanism).

The tablet 302 is also configured to deal with home automation 314, sending control signals via RF transmitter 50.

As with the above embodiment of Figure 4, the tablet includes a Bluetooth 49 facility arranged to communicate with medical devices (e.g. blood pressure monitors, heart rate monitors, etc.) 315 and accessibility devices 316.

The tablet also includes a DECT facility 317 for receiving signals from the DECT cordless phone 303. Tablet 302 then processes the telephone signals and facilitates telephone communication via GSM 51 or ADSL 55.

The tablet 302 therefore includes the majority of functionality of the management apparatus 300. The dock provides some functionality from the hard buttons 304 including the hard help button 320. Signals from these buttons are processed by the MCU 90 and then transmitted onto the tablet 302 by RF transmitter 321 in the dock 301. All alarms therefore go via the tablet 302. Communications signals can be received by the dock e.g. from the ADSL modem and transmitted to the tablet 302 for processing.

The dock includes a power management system 331 which is arranged to feed power to the tablet 302 (when the tablet is docked) and also to the battery 332 of the cordless phone 303, when the cordless phone is docked to the battery power point.

Status led LED's 333 are provided to show the status of the apparatus.

The dock 302 also includes the amplifier 335 for the dock speakers and microphone. In this embodiment, audio is provided by the dock 301 only when the tablet 302 is docked.

Referring to Figure 28, an alternative architecture is illustrated for the embodiment of Figure 26. In this alternative architecture, the dock 301 has more functionality than the embodiment of Figure 27. The tablet 302 has less functionality. Essentially, in the embodiment of Figure 28, the dock 301 comprises an RF receive and RF transmitter 340 arranged to receive communications from alarms and also arranged to control home automation. PERS alarms 310, 311 are therefore routed via the dock, as are other security alarms 312, 313. The dock may transmit directly via the ADSL modem so that the alarms do not even have to go via the tablet. They can go via the tablet GSM 51, however, via WiFi transmission 65 from the dock 301 to the tablet 302. Home automation 314 is controlled via the dock 301. Home automation may be (as discussed above) controlled remotely, or may be controlled via the patient interface via the tablet 302 communicating with the dock 301.

In this embodiment, the dock 301 also includes a Bluetooth facility 341 for a further communication pathway with the tablet 302.

Dock audio will be through the dock connector or via WiFi or BlueTooth.

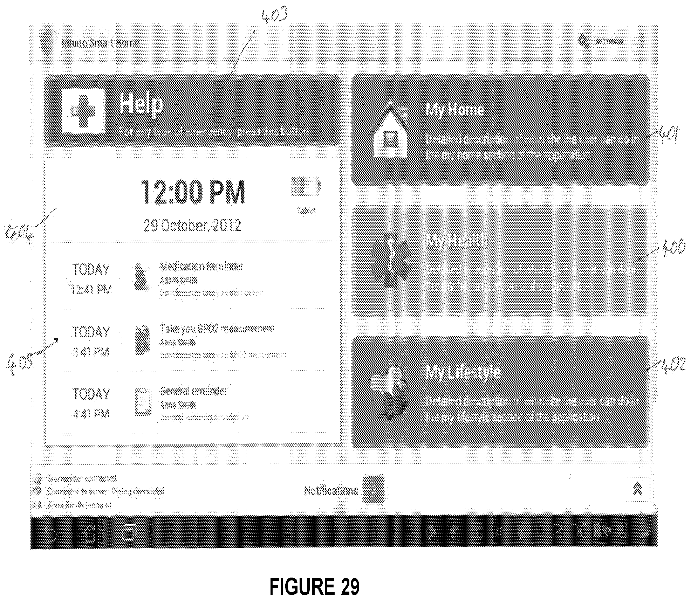

Figure 29 illustrates a "Home" screen that may be displayed by the tablet of the embodiment of Figure 26. It can be seen that the user can select a "My Help" menu 400, "My Home" menu 401 and a "My Lifestyle" 402 menu. There is also a soft "Help" button 403, and information on time 404 and reminders, such as medication reminders 405 that can be used to remind the patient that they need to take medications, as well as other general reminders.

The My Lifestyle section 402 may facilitate access to social events (that in some cases may be provided by the village administrator, or by the monitoring system 11, or by other means).

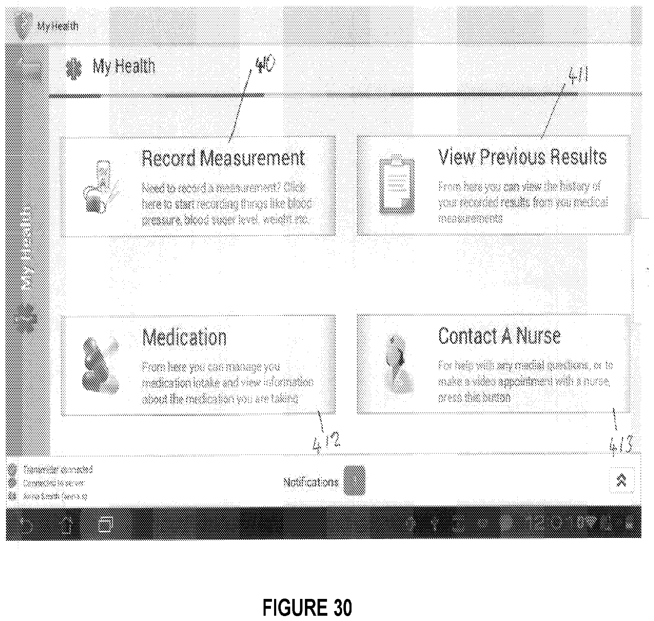

Figure 30 is a screen underneath the "My Help" menu showing various options that may be presented to the user, such as:
- "Record Measurement" 410, where the patient may wish to record a health measurement;
- The patient can also "View Previous Results" of their medical history, reference numeral 411;
- There is a "Medication" menu 412 where a patient can manage medication intake and view information about the medication the patient is taking. The medication menu may even provide a convenient conduit for the comparison and advertising of available brands of medication;
- There is also an option 413 to "Contact a Nurse" via the remote monitoring system 11.

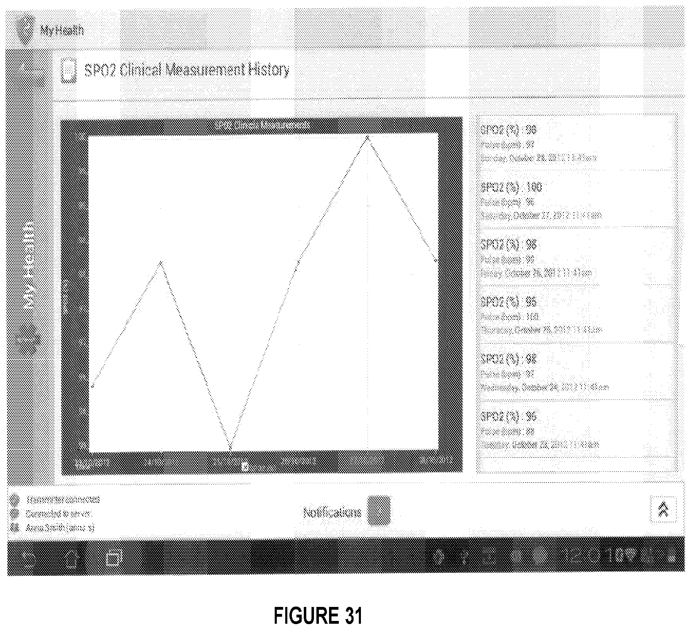

Figure 31 is an example screen under the "View Previous Results" menu, showing clinical measurement history, in this example for SP02. Any other results may be displayed under this menu of any clinical or other medical measurements.

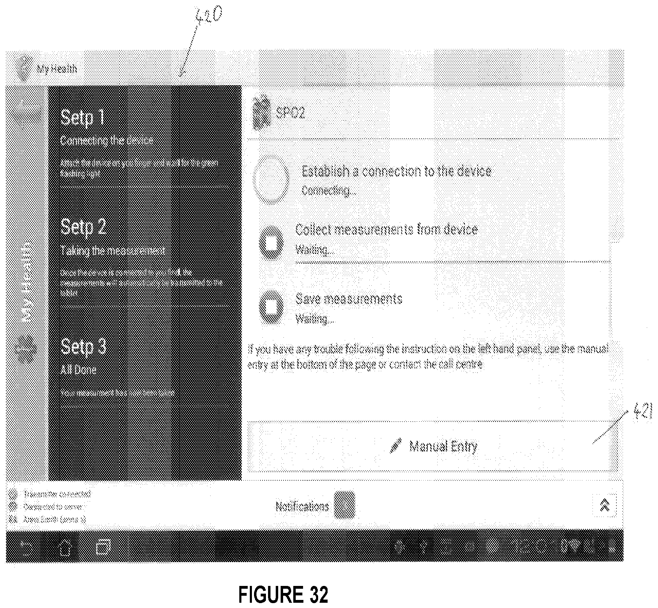

Figure 32 shows an example screen shot of a Record Measurement item, in this case connection and measure of SP02 from an SP02 device. The menu leads the patient through taking the measurement and connecting the device 420. If the device cannot be detected for some reason, then there is a manual entry option 421.

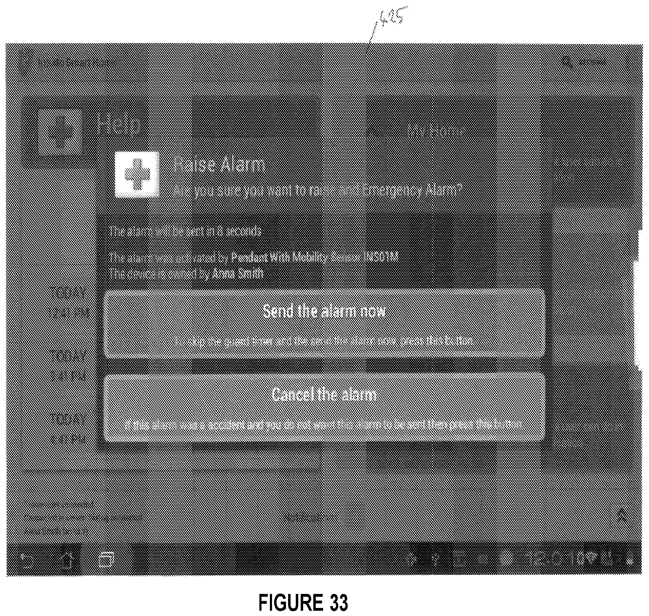

Figures 33 to 35 show screens relating to raising of an alarm and subsequent video connection with the monitoring system 11.

Figure 33 shows a check screen, just in case the alarm button was pressed by mistake, reference numeral 425.

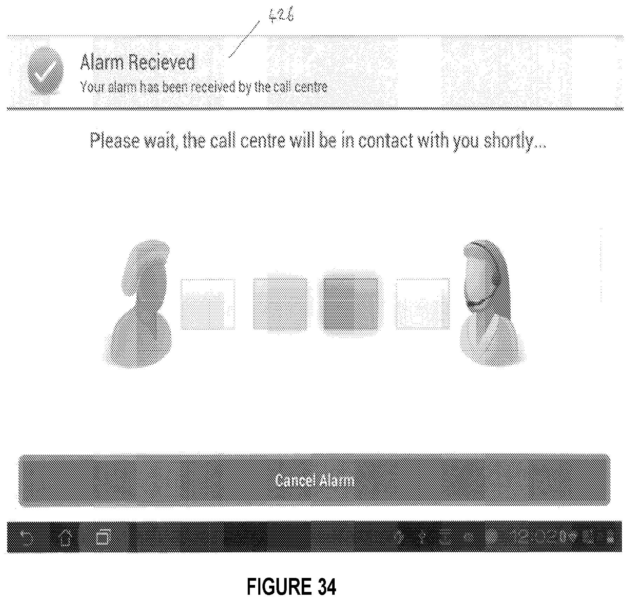

Figure 34 shows the call waiting screen, reassuring the patient that the alarm has been received (reference numeral 426) and that they will be contacted shortly.

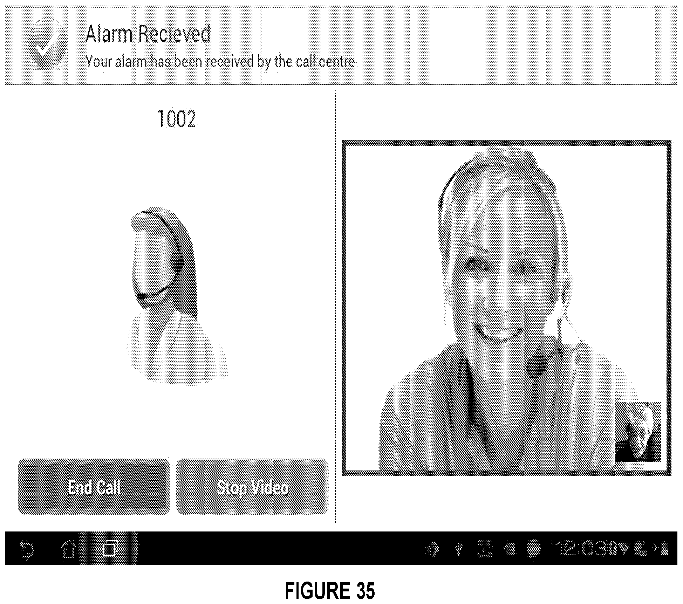

Figure 35 shows contact and video call with an operative.

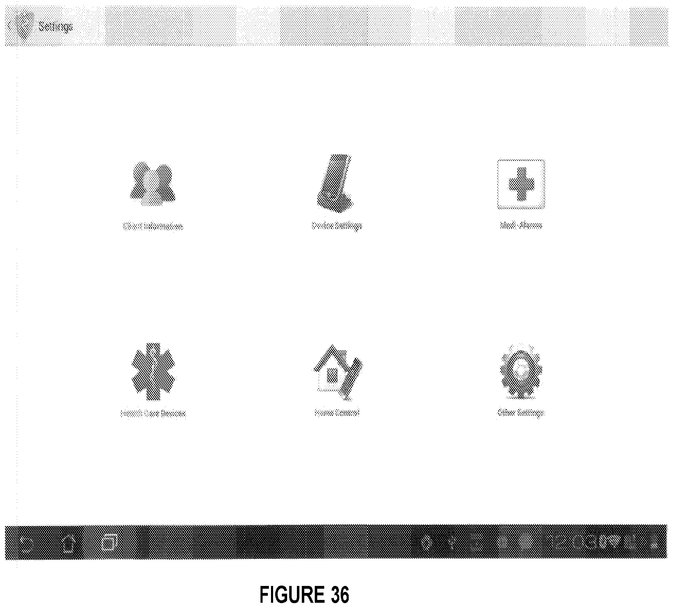

Figure 36 illustrates an example Settings menu.

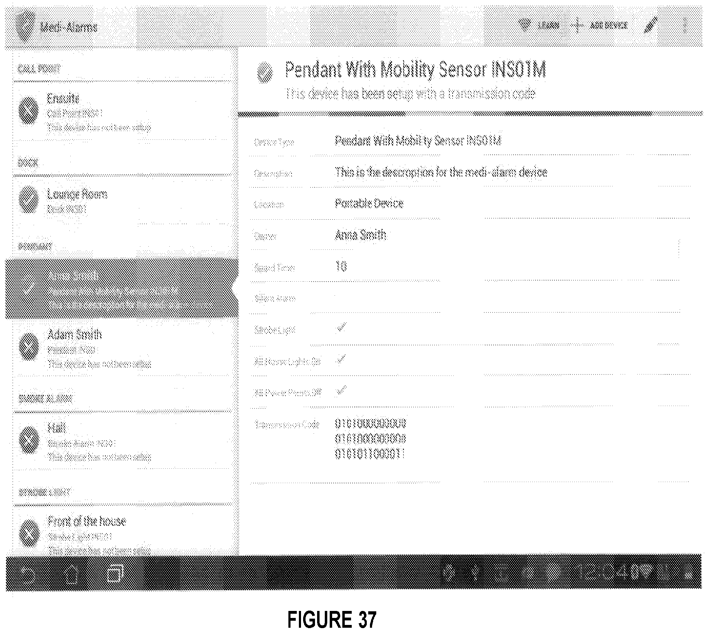

Figure 37 shows how a sensor (in this case a mobility sensor) may be set up, either by the patient, or more likely by an operative setting up the system.

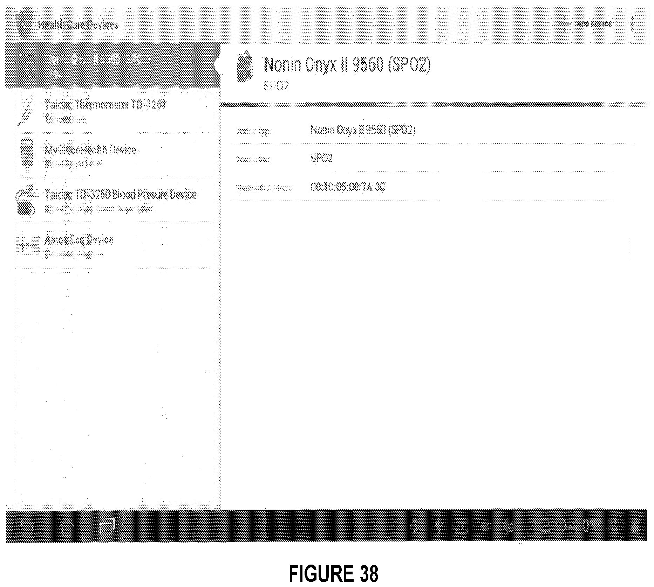

Figure 38 shows set up of a health care device (in this case SP02 sensor).

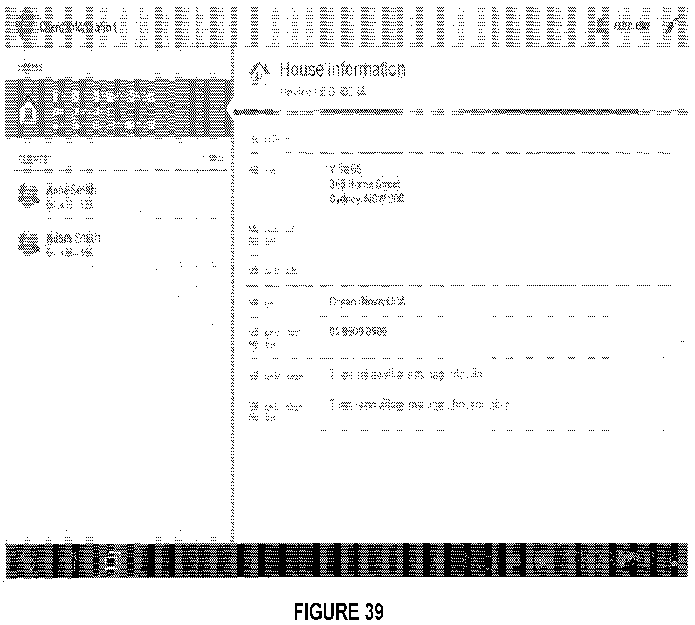

Figure 39 shows a screen where the client can enter their information about their house.

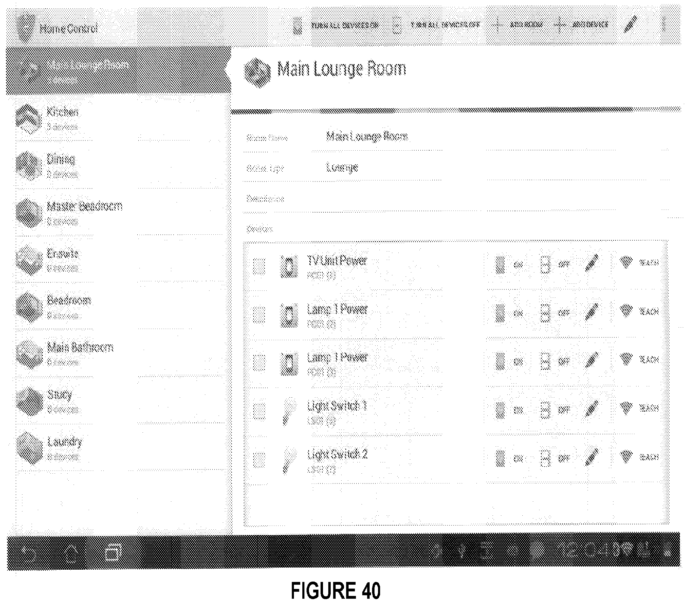

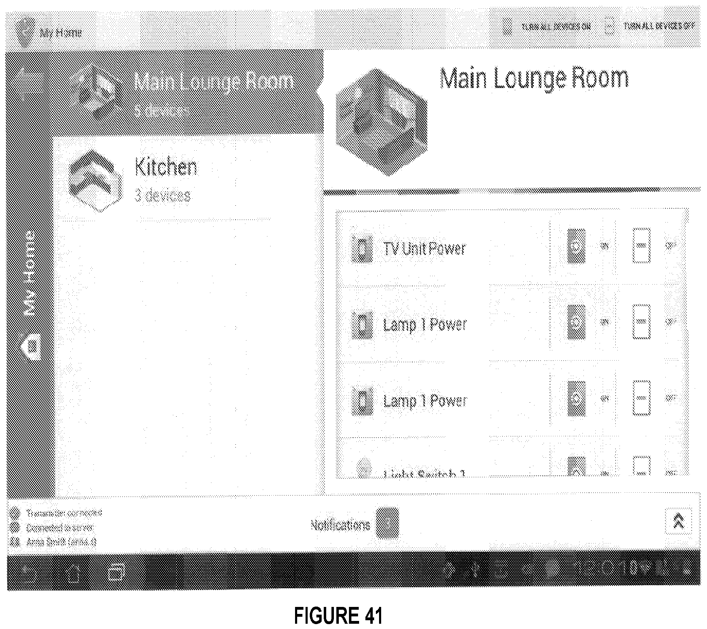

Figures 40 and 41 show screens facilitating control of devices in the house. These may be controlled by the patient. Equivalent screens may be produced by monitoring system 11 for remote control by an operative.

It will be appreciated that the screenshots in the drawings are sample screen shots only, and for the functionality of the apparatus other screens may be utilised additionally or alternatively.

The management apparatus of the above embodiments are generally intended for use in the home/residence of the patient. In embodiments, a single apparatus may be used by more than one person. For example a husband and wife living in a care village may each have separate logins to a single apparatus, providing their health care and other functionality.

In another embodiment, a management apparatus may be arranged for multiple users. For example, the apparatus may be configured as a "kiosk" in a community for access by all members of the community, each member having a separate login. Login could be via password, fingerprint (or any other biometric) RFID tag/card or combinations of these.

Thus embodiment enables each person to enter video calls and monitor their health signs (e.g. blood pressure). They can also have video calls with medical operatives, such as nurses. Also be promptedy (when they are logged in) to take their medication, etc. The device could also have a functionality of providing social information on the community, for example. The device could also be configured to receive alarms and transmit them to the remote location.

One of the main problems with health care, particularly in demographics where there is an aging population, is the expense of health care. The requirement for many operatives, such as nurses, to attend at various locations to care for is very expensive. It often results in limited care, or bringing patients into care centres, such as hospitals earlier than would normally be the case, because of lack of resources meaning.

It is envisaged that embodiments of the present invention have the advantage that care can be delivered more efficiently and cost effectively, meaning that patients get good care, and also that the care can be delivered remotely so that they can be maintained at their place of residence for longer than would normally be the case.

Because the management apparatus enables a channel between remote monitoring systems (such as monitoring system 11) and the management apparatus, this facilitates close monitoring of patient welfare. For example, if there is an alarm incident in a patient's home, a medical alarm actuated by the patient, or a health device showing that the patient's parameters are outside the required limits, then an operative of system 11 first of all can find out what is going on in the home by first of all opening an audio channel and trying to speak to the patient. If audio is not successful, they can then implement video (via the device camera) to see what is happening with the patient.

In addition, they are able to monitor and control home devices e.g. lights, door locks. This therefore enables a great deal of action to be taken with a patient without it being necessary to send out an operative. Of course, an operative may need to be sent out, but many options are available til then. Further, the fact that there can be a channel opened to the patient, means that a response can be rapid as compared with sending out a medical operative.

Systems in accordance with the present invention will be able to deliver better service in a more cost effective manner.

In the above embodiments, the tablet's components have been designed particularly for the management apparatus application. In some embodiments, it is possible that an "off the shelf" tablet computer can be programmed to give some of the functionality discussed above.

In the above embodiment, the cordless phone is charged via the dock. A separate phone charger may also be provided for the cordless phone, outside the dock.

In the above embodiment, security and health signals will go back to the monitoring system 11. In other embodiments, security may go to a separate system and health go to the monitoring system 11.

It will be appreciated by a person skilled in the art that numerous variations and/or modifications may be made to the present invention as shown in the specific embodiment without departing from the scope of the invention as broadly described. The present embodiment is, therefore, to be considered in all respects to be illustrative and not restrictive.

## Claims

1. A health and security management apparatus (3,300) for facilitating the management of health and security of a patient and their home, the management apparatus comprising a processing device having a processor (93), memory, a remote communications interface (4, 5, 50, 340, 51, 55) for communicating with a remote monitoring system which is arranged for communications with a plurality of the health and security apparatus, a local communications interface (50, 340, 53, 49, 72, 75) for communication with devices locally, a patient interface for operation by the patient, the patient interface comprising a display screen for display and input means for patient input, a health monitoring arrangement being arranged to obtain health data on patient health, and arranged to transmit the data via the remote communication interface to the remote monitoring system (11), a security monitoring arrangement arranged to obtain data on patient security, including personal alarm data and home security data, including obtaining the patient security data via the local communications interface from security devices (301, 311, 312, 303) locally, and arranged to transmit the data via the remote communications interface to the remote monitoring system, a home automation arrangement (30, 314) arranged to produce home automation data to control home automation devices (31, 32) in the patient's home, the home automation data being transmitted to the home automation devices via the local communication interface, and the home automation arrangement being arranged to receive home automation data from the remote monitoring system via the remote communications interface, for control of home automation devices from the remote monitoring system, wherein the remote communication interface comprises a plurality of different communication modules (4, 5, 50, 55, 72, 340), enabling communications by different pathways, to enable redundancy of communications with the remote monitoring system , and wherein the health and security management apparatus converges the health monitoring arrangement, the security monitoring arrangement and the home automation arrangement into a single apparatus, wherein the single apparatus comprises a plurality of units arranged to be mounted to each other, and wherein the health and security management apparatus being arranged to port all health data, security data and home automation data.

2. An apparatus in accordance with Claim 1, wherein the management apparatus (3,300)is implemented by an architecture comprising a portable unit (40, 302) and a dock unit, the portable unit being arranged to be docked with the dock unit (41, 301), the portable unit being separable from the dock unit so as to be portable.

3. An apparatus in accordance with Claim 2, wherein the dock (41, 301) is arranged to support the portable unit (40, 302) in an upright position for ease of access by the patient.

4. An apparatus in accordance with Claim 2 or Claim 3, the dock unit (41, 301) comprising an enhanced audio arrangement comprising speakers and microphones, being of enhanced functionality as compared with an audio arrangement of the portable unit (40, 302).

5. An apparatus in accordance with any one of Claims 2 to 4, the dock unit comprising a power supply (120, 121) which is arranged to recharge a power supply (120, 121)of the portable unit (40, 302) when the portable unit and dock unit (41, 301) are docked.

6. An apparatus in accordance with any one of Claims 2 to 5, the dock unit (41, 301) comprising a plurality of interface elements (76, 81, 84) in the form of relatively large buttons which are relatively easy to manipulate for an old or infirm patient.

7. An apparatus in accordance with any one claims 2 to 6, wherein one of the plurality of units comprises a portable telephone (80), arranged to be docked with the apparatus, and being arranged to implement patient communications.

8. An apparatus in accordance with any one of preceding claims, wherein the communications interface further comprises a browser enabling the patient to access and communicate via the Internet and worldwide web

9. An apparatus in accordance with any one of the preceding claims, wherein the communications interface is arranged to attempt different ones of the plurality of available communications pathways, should any one of them not be operating to communicate data.

10. An apparatus in accordance with any of the preceding claims, wherein the patient interface comprises a communications interface which is arranged to enable the patient to communicate remotely.

11. An apparatus in accordance with any one of the preceding claims, wherein the patient interface is generated by a single application platform, providing a consistent interface across all facilities of the management apparatus (3, 300).

12. An apparatus in accordance with any one of the preceding claims, wherein the patient interface comprises a two-way messaging system between the remote system and the management apparatus, comprising a "widget".

13. An apparatus in accordance with any one of the preceding claims, wherein one of the plurality of units comprises a unit arranged to be supported by the apparatus in an upright position, and comprising the display screen.

14. A health management system, arranged for management of health and security of patients at a plurality of different locations, comprising a health and security management apparatus in accordance with any one of Claims 1 to 13, and a monitoring system for facilitating the management of health and security of patients at remote locations, comprising a monitoring communications system (11)for communicating with a plurality of health and security management apparatus (3,300) in accordance with any one of the preceding claims, and being arranged to receive health data on patient health and security data on security of the patient, and being arranged to provide control data automation data to the health and security management apparatus, for control of the patient's home system.

15. A health management system in accordance with Claim 14, the monitoring system being arranged to provide control data for remote control of the management apparatus (3,300).

## Patentansprüche

1. Gesundheits- und Sicherheitsmanagementvorrichtung (3, 300) zum Erleichtern des Managements von Gesundheit und Sicherheit eines Patienten und seines Heims, wobei die Managementvorrichtung eine Verarbeitungsvorrichtung umfasst, die Folgendes aufweist: einen Prozessor (93), einen Speicher, eine Fernkommunikationsschnittstelle (4, 5, 50, 340, 51, 55) zum Kommunizieren mit einem Fernüberwachungssystem, das für die Kommunikation mit mehreren der Gesundheits- und Sicherheitsmanagementvorrichtungen ausgelegt ist, eine lokale Kommunikationsschnittstelle (50, 340, 53, 49, 72, 75) zur lokalen Kommunikation mit Einrichtungen, eine Patientenschnittstelle für die Bedienung durch den Patienten, wobei die Patientenschnittstelle einen Anzeigebildschirm zur Anzeige und Eingabemittel für die Patienteneingabe umfasst, eine Gesundheitsüberwachungsanordnung, die dafür ausgelegt ist, Gesundheitsdaten über die Patientengesundheit zu erhalten, und die ausgelegt ist, die Daten über die Fernkommunikationsschnittstelle zu dem Fernüberwachungssystem (11) zu übertragen, eine Sicherheitsüberwachungsanordnung, die dafür ausgelegt ist, Daten über die Patientensicherheit zu erhalten, die persönliche Alarmdaten und Heimsicherheitsdaten enthalten, die das lokale Erhalten der Patientensicherheitsdaten über die lokale Kommunikationsschnittstelle von Sicherheitseinrichtungen (301, 311, 312, 303) enthalten, und die ausgelegt ist, die Daten über die Fernkommunikationsschnittstelle zu dem Fernüberwachungssystem zu übertragen, eine Heimautomatisierungsanordnung (30, 314), die dafür ausgelegt ist, Heimautomatisierungsdaten zu erzeugen, um Heimautomatisierungseinrichtungen (31, 32) in dem Heim des Patienten zu steuern, wobei die Heimautomatisierungsdaten über die lokale Kommunikationsschnittstelle zu den Heimautomatisierungseinrichtungen übertragen werden, und die Heimautomatisierungsanordnung dafür ausgelegt ist, über die Fernkommunikationsschnittstelle Heimautomatisierungsdaten von dem Fernüberwachungssystem zu empfangen, um Heimautomatisierungseinrichtungen von dem Fernüberwachungssystem zu steuern, wobei die Fernkommunikationsschnittstelle mehrere verschiedene Kommunikationsmodule (4, 5, 50, 55, 72, 340) umfasst, die eine Kommunikation über verschiedene Wege ermöglichen, um eine Redundanz der Kommunikation mit dem Fernüberwachungssystem zu ermöglichen, und wobei die Gesundheits- und Sicherheitsmanagementvorrichtung die Gesundheitsüberwachungsanordnung, die Sicherheitsüberwachungsanordnung und die Heimautomatisierungsanordnung zu einer einzelnen Vorrichtung zusammenführt, wobei die einzelne Vorrichtung mehrere Einheiten umfasst, die dafür ausgelegt sind, aneinander montiert zu werden, und wobei die Gesundheits- und Sicherheitsmanagementvorrichtung dafür ausgelegt ist, alle Gesundheitsdaten, Sicherheitsdaten und Heimautomatisierungsdaten zu portieren.

2. Vorrichtung nach Anspruch 1, wobei die Managementvorrichtung (3, 300) durch eine Architektur implementiert wird, die einer tragbare Einheit (40, 302) und eine Andockeinheit umfasst, wobei die tragbare Einheit dafür ausgelegt ist, an die Andockeinheit (41, 301) angedockt zu werden, wobei die tragbare Einheit von der Andockeinheit getrennt werden kann, um tragbar zu sein.

3. Vorrichtung nach Anspruch 2, wobei die Andockeinheit (41, 301) dafür ausgelegt ist, die tragbare Einheit (40, 302) zur Erleichterung des Zugriffs durch den Patienten in einer aufrechten Position zu tragen.

4. Vorrichtung nach Anspruch 2 oder Anspruch 3, wobei die Andockeinheit (41, 301) eine verbesserte Audioanordnung umfasst, die Lautsprecher und Mikrophone umfasst, die im Vergleich zu einer Audioanordnung der tragbaren Einheit (40, 302) eine verbesserte Funktionalität besitzt.

5. Vorrichtung nach einem der Ansprüche 2 bis 4, wobei die Andockeinheit eine Stromversorgung (120, 121) umfasst, die dafür ausgelegt ist, eine Stromversorgung (120, 121) der tragbaren Einheit (40, 302) wieder aufzuladen, wenn die tragbare Einheit und die Andockeinheit (41, 301) aneinander gedockt sind.

6. Vorrichtung nach einem der Ansprüche 2 bis 5, wobei die Andockeinheit (41, 301) mehrere Schnittstellenelemente (76, 81, 84) in Form von relativ großen Knöpfen umfasst, die für einen alten oder gebrechlichen Patienten relativ leicht zu handhaben sind.

7. Vorrichtung nach einem der Ansprüche 2 bis 6, wobei eine der mehreren Einheiten ein tragbares Telefon (80) umfasst, das dafür ausgelegt ist, an die Vorrichtung angedockt zu werden, und dafür ausgelegt ist, Patientenkommunikation zu implementieren.

8. Vorrichtung nach einem der vorhergehenden Ansprüche, wobei die Kommunikationsschnittstelle ferner einen Browser umfasst, der dem Patienten ermöglicht, auf das Internet und das weltweite Netz zuzugreifen und darüber zu kommunizieren.

9. Vorrichtung nach einem der vorhergehenden Ansprüche, wobei die Kommunikationsschnittstelle dafür ausgelegt ist, verschiedene der mehreren zur Verfügung stehenden Kommunikationswege auszuprobieren, sollte einer von diesen nicht in Betrieb sein, um Daten zu kommunizieren.

10. Vorrichtung nach einem der vorhergehenden Ansprüche, wobei die Patientenschnittstelle eine Kommunikationsschnittstelle umfasst, die dafür ausgelegt ist, dem Patienten zu ermöglichen, eine Fernkommunikation durchzuführen.

11. Vorrichtung nach einem der vorhergehenden Ansprüche, wobei die Patientenschnittstelle durch eine einzelne Anwendungsplattform erzeugt wird, die eine konsistente Schnittstelle über alle Funktionen der Managementvorrichtung (3, 300) bereitstellt.

12. Vorrichtung nach einem der vorhergehenden Ansprüche, wobei die Patientenschnittstelle ein Zweiwege-Benachrichtigungssystem zwischen dem Fernsystem und der Managementvorrichtung umfasst, die ein Steuerelement umfasst.

13. Vorrichtung nach einem der vorhergehenden Ansprüche, wobei eine der mehreren Einheiten eine Einheit umfasst, die dafür ausgelegt ist, von der Vorrichtung in einer aufrechten Position getragen zu werden, und die den Anzeigebildschirm umfasst.

14. Gesundheitsmanagementsystem, das für das Management der Gesundheit und Sicherheit von Patienten an mehreren verschiedenen Orten ausgelegt ist, das eine Gesundheits- und Sicherheitsmanagementvorrichtung nach einem der Ansprüche 1 bis 13, und ein Überwachungssystem zum Erleichtern des Managements der Gesundheit und Sicherheit von Patienten an entfernten Orten umfasst, das ein Überwachungskommunikationssystem (11) für das Kommunizieren mit mehreren Gesundheits- und Sicherheitsmanagementvorrichtungen (3, 300) nach einem der vorhergehenden Ansprüche umfasst und dafür ausgelegt ist, Gesundheitsdaten über die Patientengesundheit und Sicherheitsdaten über die Sicherheit des Patienten zu empfangen und dafür ausgelegt ist, der Gesundheits- und Sicherheitsmanagementvorrichtung für die Steuerung des Heimsystems des Patienten Steuerungsdaten-Automatisierungsdaten bereitzustellen.

15. Gesundheitsmanagementsystem nach Anspruch 14, wobei das Überwachungssystem dafür ausgelegt ist, Steuerungsdaten für die Fernsteuerung der Managementvorrichtung (3, 300) bereitzustellen.

## Revendications

1. Appareil de gestion de la santé et de la sécurité (3, 300) pour faciliter la gestion de la santé et de la sécurité d'un patient et de son domicile, l'appareil de gestion comprenant un dispositif de traitement ayant un processeur (93), une mémoire, une interface de communication distante (4, 5, 50, 340, 51, 55) pour communiquer avec un système de surveillance à distance qui est conçu pour communiquer avec une pluralité d'appareils de gestion de la santé et de la sécurité, une interface de communication locale (50, 340, 53, 49, 72, 75) pour communiquer avec des dispositifs locaux, une interface de patient destinée à être utilisée par le patient, l'interface de patient comprenant un écran d'affichage pour permettre un affichage et un moyen d'entrée pour permettre une entrée du patient, un agencement de surveillance de la santé conçu pour obtenir des données de santé sur la santé du patient et conçu pour transmettre les données au système de surveillance à distance (11) par l'intermédiaire de l'interface de communication distante, un agencement de surveillance de la sécurité conçu pour obtenir des données sur la sécurité du patient, y compris des données d'alarme personnelle et des données de sécurité de domicile, l'obtention consistant à obtenir les données sur la sécurité du patient par l'intermédiaire de l'interface de communication locale à partir de dispositifs de sécurité (301, 311, 312, 303) locaux, et conçu pour transmettre les données au système de surveillance à distance par l'intermédiaire de l'interface de communication distante, un agencement domotique (30, 314) conçu pour produire des données domotiques pour contrôler des dispositifs domotiques (31, 32) dans le domicile du patient, les données domotiques étant transmises aux dispositifs domotiques par l'intermédiaire de l'interface de communication locale, et l'agencement domotique étant conçu pour recevoir des données domotiques en provenance du système de surveillance à distance par l'intermédiaire de l'interface de communication distante pour contrôler les dispositifs domotiques à partir du système de surveillance à distance, l'interface de communication distance comprenant une pluralité de modules de communication différents (4, 5, 50, 55, 72, 340), permettant des communications par différentes voies, pour permettre une redondance des communications avec le système de surveillance à distance, et l'appareil de gestion de la santé et de la sécurité faisant converger l'agencement de surveillance de la santé, l'agencement de surveillance de la sécurité et l'agencement domotique en un unique appareil, l'unique appareil comprenant une pluralité d'unités conçues pour être montées l'une avec l'autre, et l'appareil de gestion de la santé et de la sécurité étant conçu pour transporter la totalité des données de santé, des données de sécurité et des données domotiques.

2. Appareil selon la revendication 1, l'appareil de gestion (3, 300) étant mis en œuvre par une architecture comprenant une unité portable (40, 302) et une unité d'accueil, l'unité portable étant conçue pour être accueillie sur l'unité d'accueil (41, 301), l'unité portable pouvant être séparée de l'unité d'accueil de manière à pouvoir être portée.

3. Appareil selon la revendication 2, dans lequel l'unité d'accueil (41, 301) est conçue pour supporter l'unité portable (40, 302) dans une position verticale pour faciliter l'accès par le patient.

4. Appareil selon la revendication 2 ou la revendication 3, dans lequel l'unité d'accueil (41, 301) comprend un agencement audio amélioré comprenant des haut-parleurs et des microphones, dont la fonctionnalité est améliorée par comparaison avec un agencement audio de l'unité portable (40, 302).

5. Appareil selon l'une quelconque des revendications 2 à 4, dans lequel l'unité d'accueil comprend une alimentation (120, 121) qui est conçue pour recharger une alimentation (120, 121) de l'unité portable (40, 302) quand l'unité portable est accueillie sur l'unité d'accueil (41, 301).

6. Appareil selon l'une quelconque des revendications 2 à 5, dans lequel l'unité d'accueil (41, 301) comprend une pluralité d'éléments d'interface (76, 81, 84) sous la forme de boutons relativement grands qui sont relativement simples à manipuler pour un patient âgé ou déficient.

7. Appareil selon l'une quelconque des revendications 2 à 6, dans lequel une unité de la pluralité d'unités comprend un téléphone portable (80), conçu pour être accueilli sur l'appareil et conçu pour mettre en œuvre des communications avec le patient.

8. Appareil selon l'une quelconque des revendications précédentes, dans lequel l'interface de communication comprend en outre un navigateur permettant au patient d'accéder à Internet et au Web mondial et de communiquer par l'intermédiaire d'Internet et du Web mondial.

9. Appareil selon l'une quelconque des revendications précédentes, dans lequel l'interface de communication est conçue pour essayer différentes voies de la pluralité de voies de communication disponibles, si l'une quelconque d'entre elles n'est pas fonctionnelle pour communiquer des données.

10. Appareil selon l'une quelconque des revendications précédentes, dans lequel l'interface de patient comprend une interface de communication qui est conçue pour permettre au patient de communiquer à distance.

11. Appareil selon l'une quelconque des revendications précédentes, dans lequel l'interface de patient est générée par une unique plateforme d'applications, fournissant une interface homogène sur toutes les installations de l'appareil de gestion (3, 300).

12. Appareil selon l'une quelconque des revendications précédentes, dans lequel l'interface de patient comprend un système de messagerie bidirectionnel entre le système distant et l'appareil de gestion, comprenant un « gadget logiciel ».

13. Appareil selon l'une quelconque des revendications précédentes, dans lequel une unité de la pluralité d'unités comprend une unité conçue pour être supportée par l'appareil dans une position verticale et comprenant l'écran d'affichage.

14. Système de gestion de la santé, conçu pour gérer la santé et la sécurité de patients situés à une pluralité d'emplacements différents, le système comprenant un appareil de gestion de la santé et de la sécurité selon l'une quelconque des revendications 1 à 13 et un système de surveillance pour faciliter la gestion de la santé et de la sécurité de patients à des emplacements distants, comprenant un système de communication de surveillance (11) pour communiquer avec une pluralité d'appareils de gestion de la santé et de la sécurité (3, 300) selon l'une quelconque des revendications précédentes et conçu pour recevoir des données de santé sur la santé du patient et des données de sécurité sur la sécurité du patient et conçu pour fournir des données de contrôle et des données domotiques à l'appareil de gestion de la santé et de la sécurité pour contrôler le système à domicile du patient.

15. Système de gestion de la santé selon la revendication 14, le système de gestion étant conçu pour fournir des données de contrôle pour le contrôle à distance de l'appareil de gestion (3, 300).
